# EUROPÄISCHE PATENTSCHRIFT

(11) **EP 0 529 022 B1**
(45) Veröffentlichungstag und Bekanntmachung des Hinweises auf die Patenterteilung: **27.09.1995**
(21) Anmeldenummer: 92905138.1
(22) Anmeldetag: 24.02.1992
(51) Int. Cl.: C07C 405/00, C07D 309/30, A61K 31/557

(54) **NEUE LEUKOTRIEN-B 4?-DERIVATE, VERFAHREN ZU IHRER HERSTELLUNG UND IHRE VERWENDUNG ALS ARZNEIMITTEL**
NEW LEUKOTRIENE B 4? DERIVATIVES, METHOD FOR PREPARING THEM AND THEIR USE AS DRUGS
NOUVEAUX DERIVES DE LEUCOTRIENES B 4?, LEUR PROCEDE DE PREPARATION ET LEUR UTILISATION COMME MEDICAMENTS

(30) Priorität: 12.03.1991 DE 4108351
(43) Veröffentlichungstag der Anmeldung: 03.03.1993
(73) Patentinhaber: SCHERING AKTIENGESELLSCHAFT, 13342 Berlin (DE)
(72) Erfinder: SKUBALLA, Werner, D-1000 Berlin 28 (DE); BUCHMANN, Bernd, D-1000 Berlin 21 (DE); HEINDL, Josef, D-1000 Berlin 38 (DE); FRÖHLICH, Wolfgang, D-1000 Berlin 31 (DE); EKERDT, Roland, D-1000 Berlin 28 (DE); GIESEN, Claudia, D-1000 Berlin 20 (DE)
(86) Internationale Anmeldenummer: DE9200151
(87) Internationale Veröffentlichungsnummer: WO9216504

(56) Entgegenhaltungen:
- EP-A- 0 103 445
- DE-A- 3 917 597
- Vth International Conference on Prostaglandins, Florence, 18-21 Mai 1982, P. BORGEAT et al.: "Inhibition of the synthesis of leukotriene B4 (LTB4) in human leukocytes by hydroxy-eicosatetraenoic acids", Seite II, siehe ganze Zusammenfassung

## Beschreibung

Aus der deutschen Offenlegungsschrift DE 39 17 597 sind LTB₄-Antagonisten, die einen Sechsring als Grundstrukturelement enthalten, bereits bekannt.
Die Erfindung betrifft neue Leukotrien-B₄-Derivate, Verfahren zu ihrer Herstellung sowie ihre Verwendung als Arzneimittel.
Leukotrien B₄ (LTB₄) wurde 1979 von B. Samuelsson und Mitarbeiter als Metabolit der Arachidonsäure entdeckt. Bei der Biosynthese wird durch das Enzym 5-Lipoxygenase zunächst als zentrales Zwischenprodukt das Leukotrien A₄ gebildet, das dann durch eine spezifische Hydrolase in das LTB₄ umgewandelt wird. 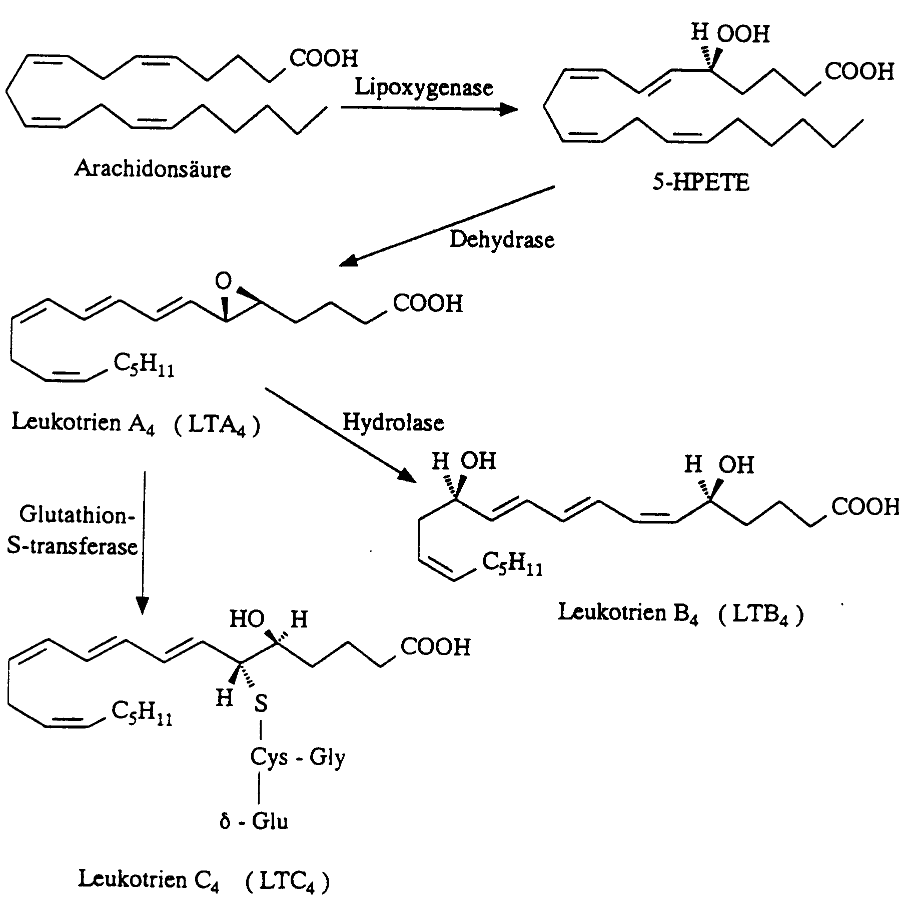
Die Nomenklatur der Leukotriene kann folgenden Arbeiten entnommen werden:
a) B. Samuelsson et al., Prostaglandins 19, 645 (1980); 17, 785 (1979).
b) C.N. Serhan et al., Prostaglandins 34, 201 (1987).

Die physiologische und insbesondere die pathophysiologische Bedeutung des Leukotrien B₄ wird in einigen neueren Arbeiten zusammengefaßt: a) The Leukotrienes, Chemistry and Biology eds. L.W. Chakrin, D.M. Bailey, Academic Press 1984. b) J.W. Gillard et al., Drugs of the Future 12, 453 (1987). c) B. Samuelsson et al., Science 237, 1171 (1987). d) C.W. Parker, Drug Development Research 10, 277 (1987). Hieraus ergibt sich, daß LTB₄ ein wichtiger Entzündungsmediator für entzündliche Erkrankungen ist, bei denen Leukocyten in das erkrankte Gewebe einwandern.
Von LTB₄ weiß man, daß es die Adhäsion von Leukozyten an die Blutgefäßwand verursacht. LTB₄ ist chemotaktisch wirksam, d.h.es löst eine gerichtete Wanderung von Leukozyten in Richtung eines Gradienten steigender Konzentration aus. Außerdem verändert es aufgrund seiner chemotaktischen Aktivität indirekt die vasculäre Permeabilität, wobei ein Synergismus mit Prostaglandin E₂ beobachtet wird. LTB₄ spielt offensichtlich bei entzündlichen, allergischen und immunologischen Prozessen eine entscheidende Rolle.

Leukotriene und insbesondere LTB₄ sind an Hauterkrankungen beteiligt, die mit entzündlichen Prozessen (erhöhte Gefäßpermeabilität und Ödembildung, Zellinfiltration), erhöhter Proliferation der Hautzellen und Juckreiz einhergehen, wie beispielsweise bei Ekzemen, Erythemen, Psoriasis, Pruritus und Akne. Pathologisch erhöhte Leukotrien-Konzentrationen sind an der Entstehung vieler Dermatitiden entweder ursächlich beteiligt, oder es besteht ein Zusammenhang zwischen der Persistenz der Dermatitiden und den Leukotrienen. Deutlich erhöhte Leukotrienkonzentrationen wurden beispielsweise in der Haut von Patienten mit Psoriasis oder atopischer Dermatitis gemessen.
Weiterhin sind Leukotriene und LTB₄ insbesondere bei Arthritis, chronischer Lungenerkrankungen (z.B. Asthma), Rhinitis und entzündlichen Darmerkrankungen beteiligt.

Antagonisten gegen LTB₄-Rezeptoren oder -Inhibitoren jener Enzyme, die an der Synthese des LTB₄ beteiligt sind, sollten als spezifische Heilmittel, besonders gegen Krankheiten, die mit Entzündungen und allergischen Reaktionen einhergehen, wirksam sein.

Neben therapeutischen Möglichkeiten, die sich aus einer Antagonisierung der LTB₄-Wirkung mit LTB₄-Analoga ableiten lassen, konnte auch die Nützlichkeit und potentielle Anwendung von Leukotrien B₄-Agonisten zur Behandlung von Pilzerkrankungen der Haut gezeigt werden (H. Katayama, Prostaglandins 34, 797 (1988)).

Die Erfindung betrifft Leukotrien-B₄-Derivate der Formel I, 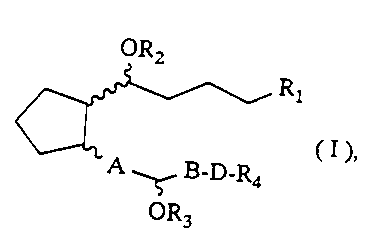
worin
- R¹: CH2OH, CH₃, CF₃, COOR⁵, CONR⁶R⁷, oder
- R¹: gemeinsam mit R² eine Carbonylgruppe bedeutet.
- R² und R³: gleich oder verschieden H oder ein organischer Säurerest mit 1-15C-Atomen darstellen,
- R⁴: H, C₁-C₁₀ gegebenenfalls einfach oder mehrfach durch Chlor oder Brom substituierter Alkyl, C₃-C₁₀-Cycloalkyl, gegebenenfalls unabhängig voneinander einfach oder mehrfach durch Chlor, Brom, Phenyl, C₁-C₄-Alkyl, C₁-C₄-Alkoxy, Fluormethyl, Chlormethyl, Trifluormethyl, Carboxy oder Hydroxy substituierter C₆-C₁₀-Arylrest oder ein 5-6 gliedriger aromatischer heterocyclischer Ring mit wenigstens 1 Heteroatom symbolisieren,
- R⁵: Wasserstoff, C₁-C₁₀ Alkyl, C₃-C₁₀ Cycloalkyl, gegebenenfalls durch 1-3 Chlor, Brom, Phenyl, C₁-C₄-Alkyl, C₁-C₄-Alkoxy, Fluormethyl, Chlormethyl, Trifluormethyl, Carboxy oder Hydroxy substituierter C₆-C₁₀ Arylrest, CH₂-CO-(C₆-C₁₀) Aryl oder ein 5-6 gliedriger Ring mit wenigstens 1 Heteroatom bedeutet,
- A: eine trans,trans-CH=CH-CH=CH, eine -CH₂CH₂-CH=CH- oder eine Tetramethylengruppe,
- B: eine C₁-C₁₀ grad- oder verzweigtkettige Alkylengruppe, die gegebenenfalls durch Fluor substituiert sein kann oder die Gruppe 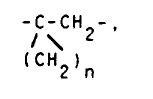 symbolisiert,
- D: eine Direktbindung, Sauerstoff, Schwefel, -C≡C-,-CH=CR⁸, oder gemeinsam mit
- B: auch eine Direktbindung bedeuten können,
- R⁶ und R⁷: gleich oder verschieden sind und H oder C₁-C₄-Alkyl oder R⁷ H und R⁶ C₁-C₁₀-Alkanoyl oder C₁-C₁₀ Alkansulfonyl darstellen,
- R⁸: H, C₁-C₅ Alkyl, Chlor, Brom bedeutet,
- n: 3-5 ist sowie, wenn R⁵ Wasserstoff bedeutet, deren Salze mit physiologisch verträglichen Basen und deren Cyclodextrinclathrate.

Die Gruppen OR² und OR³ können α- oder β-ständig sein. Die Formel I umfaßt sowohl Racemate als auch die möglichen reinen Diastereomeren und Enantiomeren.

Als Alkylgruppen R⁵ kommen gerad- oder verzweigtkettige Alkylgruppen mit 1-10 C-Atomen in Betracht, wie beispielsweise Methyl, Ethyl, Propyl, Butyl, Isobutyl, tert.-Butyl, Pentyl, Neopentyl, Hexyl, Heptyl, Decyl. Die Alkylgruppen R⁵ können gegebenenfalls ein- bis mehrfach substituiert sein durch Halogenatome, Alkoxygruppen, gegebenenfalls substituierte Aryl- bzw. Aroylgruppen mit 6-10 C-Atomen (Substitution s. unter Aryl R⁵), Dialkylamino und Trialkylammonium mit 1-4 C-Atomen im Alkyl-Teil, wobei die einfache Substitution bevorzugt sein soll. Als Substituenten seien beispielsweise genannt Fluor, Chlor oder Brom, Phenyl, Dimethylamino, Diethylamino, Methoxy, Ethoxy. Als bevorzugte Alkylgruppen R⁵ sind solche mit 1-4 C-Atomen zu nennen.

Die Cycloalkylgruppe R⁵ kann im Ring 3-10, vorzugsweise 5 und 6 Kohlenstoffatome enthalten. Die Ringe können durch Alkylgruppen mit 1-4 Kohlenstoffatomen substituiert sein. Beispielsweise seien genannt Cyclopentyl-, Cyclohexyl, Methylcyclohexyl.

Als Arylgruppen R⁵ kommen sowohl substituierte wie auch unsubstituierte Arylgruppen mit 6-10 C-Atomen in Betracht, wie beispielsweise Phenyl, 1-Naphthyl und 2-Naphthyl, die jeweils substituiert sein können durch 1-3 Halogenatome (F, Cl, Br), eine Phenylgruppe, 1-3 Alkylgruppen mit jeweils 1-4 C-Atomen, eine Chlormethyl-, Fluormethyl-, Trifluormethyl,-Carboxyl,-Hydroxy- oder Alkoxygruppe mit 1-4 C-Atomen. Bevorzugte Substituenten in 3- und 4-Stellung am Phenylring sind zum Beispiel Fluor, Chlor, Alkoxy oder Trifluormethyl, in 4-Stellung dagegen Hydroxy.

Als heterocyclische Gruppen R⁵ kommen 5- und 6-gliedrige aromatische Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3-Furyl, 3-Thienyl, 2-Tetrazolyl u.a.

Als Säurerest R⁶ kommen physiologisch verträgliche Säurereste in Frage. Bevorzugte Säuren sind organische Carbonsäuren und Sulfonsäuren mit 1-15 Kohlenstoff atomen, die der aliphatischen, cycloaliphatischen, aromatischen, aromatisch-aliphatischen und heterocyclischen Reihe angehören. Diese Säuren können gesättigte, ungesättigte und/oder mehrbasisch und/oder in üblicher Weise substituiert sein. Als Beispiele für die Substituenten seien C₁₋₄-Alkyl-, Hydroxy-, C₁₋₄-Alkoxy, Oxo- oder Aminogruppen oder Halogenatome (F, Cl, Br) erwähnt. Beispielsweise seien folgende Carbonsäuren genannt: Ameisensäure, Essigsäure, Propionsäure, Buttersäure, Isobuttersäure, Valeriansäure, Isovaleriansäure, Capronsäure, Onanthsäure, Caprylsäure, Pelargonsäure, Caprinsäure, Undecylsäure, Laurinsäure, Tridecylsäure, Myristinsäure, Pentadecylsäure, Trimethylessigsäure, Diethylessigsäure, tert.-Butylessigsäure, Cyclopropylessigsäure, Cyclopentylessigsäure, Cyclohexylessigsäure, Cyclopropancarbonsäure, Cyclohexancarbonsäure, Phenylessigsäure, Phenoxyesigsäure, Methoxyessigsäure, Ethoxyessigsäure, Mono-, Di- und Trichloressigsäure, Aminoessigsäure, Diethylaminoessigsäure, Piperidinoessigsäure, Morpholinoessigsäure, Milchsäure, Bernsteinsäure, Adipinsäure, Benzoesäure, mit Halogen (F, Cl, Br) oder Trifluormethyl-, Hydroxy, C₁₋₄-Alkoxy- oder Carboxy-Gruppen substituierte Benzoesäuren, Nikotinsäure, Isonikotinsäure, Furan-2-carbonsäure, Cyclopentylpropionsäure. Als besonders bevorzugte Acylreste und Alkansulfonylreste werden solche mit bis zu 10 Kohlenstoffatomen betrachtet. Als Sulfonsäuren kommen beispielsweise Methansulfonsäure, Ethansulfonsäure, Isopropansulfonsäure, β-Chlorethansulfonsäure, Butansulfonsäure, Cyclopentansulfonsäure, Cyclohexansulfonsäure, Benzolsulfonsäure, p-Toluolsulfonsäure, p-Chlorbenzolsulfonsäure, N,N-Dimethylaminosulfonsäure, N,N-Diethylaminosulfonsäure, N,N-Bis-(β-chlorethyl)-aminosulfonsäure, N,N-Diisobutylaminosulfonsäure, N,N-Dibutylaminosulfonsäure, Pyrrolidino-, Piperidino-, Piperazino-, N-Methylpiperazino- und Morpholinosulfonsäure in Frage.

Als Alkylgruppen R⁴ kommen gerad- und verzweigtkettige, gesättigte und ungesättigte Alkylreste, vorzugsweise gesättigte, mit 1-14, insbesondere 1-10 C-Atomen, in Frage, die gegebenenfalls durch gegebenenfalls substituiertes Phenyl (Substitution s. unter Aryl R⁵) substituiert sein können. Beispielsweise genannt seien Methyl-, Ethyl-, Propyl-, Butyl-, Isobutyl-, tert.,-Butyl-, Pentyl-, Hexyl-, Heptyl-, Octyl-, Butenyl-, Isobutenyl-, Propenyl-, Pentenyl-, Benzyl-, m- und p-Chlorbenzylgruppen. Sind die Alkylgruppen R⁴ Halogen-substituiert, kommen als Halogene Fluor, Chlor und Brom in Frage.

Als Beispiel für Halogen-substituierte Alkylgruppen R⁴ kommen Alkyle mit terminalen Trifluormethylgruppen in Betracht.

Die Cycloalkylgruppe R⁴ kann im Ring 3-10, vorzugsweise 3-6 Kohlenstoffatome enthalten. Die Ringe können durch Alkylgruppen mit 1-4 Kohlenstoffatomen substituiert sein. Beispielsweise genannt seinen Cyclopropyl, Cyclobutyl, Cyclopentyl, Cyclohexyl, Methyl-cyclohexyl.

Als substituierte bzw. unsubstituierte Arylgruppen R⁴ kommen beispielsweise in Betracht: Phenyl, 1-Naphthyl und 2-Naphthyl, die jeweils substituiert sein können durch 1-3 Halogenatome, eine Phenylgruppe, 1-3 Alkylgruppen mit jeweils 1-4 C-Atomen eine Chlormethyl-, Fluormethyl-, Trifluormethyl-, Carboxyl-, C₁-C₄Alkoxy- oder Hydroxygruppe. Bevorzugt ist die Substitution in 3- und 4-Stellung am Phenylring zum Beispiel durch Fluor, Chlor, Alkoxy oder Trifluormethyl oder in 4-Stellung durch Hydroxy.

Als heterocyclische aromatische Gruppen R⁴ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3-Furyl, 3-Thienyl, u.a.

Als Alkylengruppe B kommen geradkettige oder verzweigtkettige, gesättigte oder ungesättigte Alkylenreste, vorzugsweise gesättigte mit 1-10, insbesondere mit 1-5 C-Atomen, in Frage, die gegebenenfalls durch Fluoratome substituiert sein können. Beispielsweise seien genannt: Methylen, Fluormethylen, Difluormethylen, Ethylen, 1,2-Propylen, Ethylethylen, Trimethylen, Tetramethylen, Pentamethylen, 1.2-Difluorethylen, 1-Fluorethylen, 1-Methyltetramethylen, 1-Methyl-tri-methylen, 1-Methylenethylen, 1-Methylen-tetramethylen.
Die Alkylengruppe B kann weiterhin die Gruppe 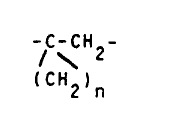
darstellen, wobei n=3-5, bevorzugt 4-5 bedeutet.

Als Säurereste R² und R³ kommen physiologisch verträgliche Säurereste in Frage. Bevorzugte Säuren sind organische Carbonsäuren und Sulfonsäuren mit 1-15 Kohlenstoffatomen, die der aliphatischen, cyclo-aliphatischen, aromatischen, aromatisch-aliphatischen oder heterocyclischen Reihe angehören. Diese Säuren können gesättigt, ungesättigt und/oder mehrbasisch und/oder in üblicher Weise substituiert sein. Als Beispiele für die Substituenten seien C₁₋₄-Alkyl-, Hydroxy-, C₁₋₄-Alkoxy, Oxo- oder Aminogruppen oder Halogenatome (F, Cl, Br) erwähnt.

Beispielsweise seien folgende Carbonsäuren genannt: Ameisensäure, Essigsäure, Propionsäure, Buttersäure, Isobuttersäure, Valeriansäure, Isovaleriansäure, Capronsäure, Onanthsäure, Caprylsäure, Pelargonsäure, Caprinsäure, Undecylsäure, Laurinsäure, Tridecylsäure, Myristinsäure, Pentadecylsäure, Trimethylessigsäure, Diethylessigsäure, tert.-Butylessigsäure, Cyclopentylessigsäure, Cyclohexylessigsäure, Cyclohexancarbonsäure, Phenylessigsäure, Phenoxyesigsäure, Methoxyessigsäure, Ethoxyessigsäure, Mono-, Di- und Trichloressigsäure, Aminoessigsäure, Diethylaminoessigsäure, Piperidinoessigsäure, Morpholinoessigsäure, Milchsäure, Bernsteinsäure, Adipinsäure, Benzoesaure, mit Halogen-(F, Cl, Br), Trifluormethyl-, Hydroxy-, C₁₋₄-Alkoxy- oder Carboxy-Gruppen substituierte Benzoesäuren, Nikotinsäure, Isonikotinsäure, Furan-2-carbonsäure, Cyclopentylpropionsäure. Als besonders bevorzugte Säurereste R² und R³ werden Acylreste mit bis zu 10 Kohlenstoffatomen betrachtet.

Die Alkylreste R⁶ und R⁷ sind gradkettige oder verzweigte Alkylreste, insbesondere geradkettige wie zum Beispiel Methyl, Ethyl, Propyl, Butyl, Pentyl, Hexyl, besonders bevorzugt Methyl.

R⁸ als C₁₋₅-Alkyl bedeutet geradkettige oder verzweigtkettige Alkylreste wie sie für R⁴ bzw. R⁵ bereits genannt wurden. Bevorzugte Alkylreste R⁸ sind Methyl, Ethyl, Propyl und Isopropyl.

Zur Salzbildung sind anorganische und organische Basen geeignet, wie sie dem Fachmann zur Bildung physiologisch verträglicher Salze bekannt sind. Beispielsweise seien genannt Alkalihydroxide, wie Natrium- und Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin usw.

Bevorzugte Verbindungen der vorliegenden Erfindung sind Verbindungen der Formel I, wobei die Reste die folgende Bedeutung haben:
- R¹: ist CH₂OH, COOR⁵ mit R⁵ in der Bedeutung eines Wasserstoffatoms, eines Alkylrestes mit 1-10 C-Atomen, eines Cycloalkylrestes mit 5-6 C-Atomen, eines gegebenenfalls durch 1-2 Chlor, Brom, Phenyl, C₁₋₄-Alkyl, C₁₋₄-Alkoxy, Chlormethyl, Fluormethyl, Trifluormethyl, Carboxy oder Hydroxy substituierten Phenylrestes
- A: ist eine trans, trans-CH=CH-CH=CH- oder Tetramethylengruppe;
- B: ist eine geradkettige oder verzweigtkettige gesättigte oder unge-Alkylengruppe mit bis zu 10 C-Atomen, die gegebenenfalls durch Fluor sub- stituiert sein kann, oder die Gruppe 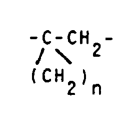 mit n=3-5;
- D: ist eine Direktbindung, Sauerstoff, Schwefel, eine -C≡C-Gruppe oder eine -CH=CR⁸-Gruppe mit R⁸ als Wasserstoff, C₁₋₅-Alkyl, Chlor oder Brom;
- B und D: sind gemeinsam eine Direktbindung;
- R² und R³: sind gleich oder verschieden und bedeuten Wasserstoff oder einen organischen Säurerest mit 1-15 C-Atomen;
- R¹ und R²: sind gemeinsam eine Carbonylgruppe;
- R⁴: ist ein Wasserstoffatom, C₁₋₁₀-Alkyl, Cycloalkyl mit 5-6 C-Atomen , ein gegebenenfalls durch 1-2 Chlor, Brom, Phenyl, C₁₋₄-Alkyl, C₁₋₄-Alkoxy, Chlormethyl, Fluormethyl, Trifluormethyl, Carboxy oder Hydroxy substituierter Phenylrest und falls
- R⁵: ein Wasserstoffatom bedeutet, deren Salze mit physiologisch verträglichen Basen und deren Cyclodextrinclathrate.

Besonders bevorzugte Verbindungen der vorliegenden Erfindung sind Verbindungen der Formel I, wobei die Reste die folgende Bedeutung haben:
- R¹: ist CH₂OH, COOR⁵ mit R⁵ in der Bedeutung eines Wasserstoffatoms, eines Alkylrestes mit 1-4 C-Atomen;
- A: ist eine trans, trans-CH=CH-CH=CH- oder Tetramethylengruppe;
- B: ist eine geradkettige oder verzweigtkettige Alkylengruppe mit bis zu 5 C-Atomen;
- D: ist eine Direktbindung oder eine -C≡C-Gruppe oder eine -CH=CR⁸-Gruppe mit R⁸ als Wasserstoff oder C₁₋₅-Alkyl;
- B und D: sind gemeinsam eine Direktbindung;
- R² und R³: sind gleich oder verschieden und bedeuten Wasserstoff oder einen organischen Säurerest mit 1-6 C-Atomen;
- R¹ und R²: sind gemeinsam eine Carbonylgruppe;
- R⁴: ist ein Wasserstoffatom oder C₁₋₁₀-Alkyl und falls R⁵ ein Wasserstoffatom bedeutet, deren Salze mit physiologisch verträglichen Basen und deren Cyclodextrinclathrate.

Außerdem betrifft die Erfindung ein Verfahren zur Herstellung der erfindungsgemäßen Verbindungen der Formel I, das dadurch gekennzeichnet ist, daß man einen Aldehyd der Formel II 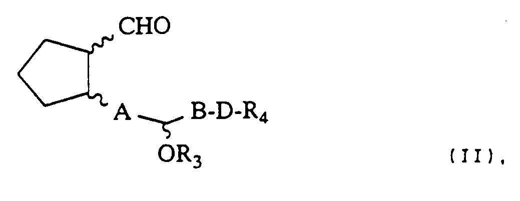
worin A, B, D, R³ und R⁴ die oben angegebene Bedeutung haben, gegebenenfalls nach Schutz freier Hydroxygruppen mit einer magnesiumorganischen Verbindung der Formel III,

X-Mg-CH₂-CH₂-CH₂-CH₂-R⁹ (III),

worin X Chlor, Brom oder Iod und R⁹ -CH₃, CF₃ oder -OR¹⁰ darstellt, worin R₁₀ einen leicht abspaltbaren Etherrest bedeuten, umsetzt und gegebenenfalls anschließend in beliebiger Reihenfolge Isomere trennt, geschützte Hydroxygruppen freisetzt und/oder eine freie Hydroxygruppe verestert und/oder die 1-Hydroxygruppe zur Carbonsäure oxidiert und/oder Doppelbindungen hydriert und/oder eine veresterte Carboxylgruppe (R¹=COOR⁵) verseift und/oder reduziert und/oder eine Carboxylgruppe (R⁵=H) verestert und/oder eine freie Carboxylgruppe (R⁵=H) in ein Amid (R¹=CONHR⁶R⁷) überführt oder eine Carboxylgruppe mit einer physiologisch verträglichen Base in ein Salz überführt.

Als Etherreste R⁹ in der Verbindung der Formel III kommen die dem Fachmann geläufigen Reste in Betracht. Bevorzugt sind leicht abspaltbare Etherreste, wie beispielsweise Dimethyl-tert.-butylsilyl, Trimethylsilyl-, Tribenzylsilyl-, Diphenyl-tert.-butylsilyl-, Tetrahydropyranyl-, Tetrahydrofuranyl- und α-Ethoxyethyl, um nur einige zu nennen.

Die Umsetzung der Verbindung der Formel II mit einer metallorganischen Verbindung der Formel III erfolgt in an sich bekannter Weise in einem inerten Lösungsmittel oder Lösungsmittelgemisch, wie zum Beispiel Diethylether, Tetrahydrofuran, Dioxan, Toluol, Dimethoxyethan, vorzugsweise Diethylether oder Tetrahydrofuran. Die Reaktion wird bei Temperaturen zwischen -100 °C und 60 °C, vorzugsweise bei -78 °C bis 0 °C durchgeführt.

Die Herstellung der für diese Umsetzung benötigten Verbindung der Formel III erfolgt durch Reaktion des entsprechenden Hydroxyhalogenids durch Veretherung mit Dihydropyran und p-Toluolsulfonsäure und anschließende Umsetzung mit Magnesium.

Die Reduktion zu den Verbindungen der Formel I mit R¹ in der Bedeutung einer CH₂OH-Gruppe wird mit einem für die Reduktion von Estern oder Carbonsäuren geeigneten Reduktionsmittel wie beispielsweise Lithiumaluminiumhydrid, Diisobutylaluminiumhydrid usw. durchgeführt. Als Lösungsmittel kommen Diethylether, Tetrahydrofuran, Dimethoxyethan, Toluol usw. in Frage. Die Reduktion wird bei Temperaturen von -30 °C bis zur Siedetemperatur des verwendeteten Lösungsmittels, vorzugsweise 0 °C bis 30 °C vorgenommen.

Die Veresterung der Alkohole der Formel I (R² = H und/oder R³ = H) erfolgt in an sich bekannter Weise. Zum Beispiel erfolgt die Veresterung dadurch, daß man ein Säurederivat, vorzugsweise ein Säurehalogenid oder Säureanhydrid, in Gegenwart einer Base wie beispielsweise NaH, Pyridin, Triethylamin, Tributylamin oder 4-Dimethylaminopyridin mit einem Alkohol der Formel I umsetzt. Die Umsetzung kann ohne Lösungsmittel oder in einem inerten Lösungsmittel, vorzugsweise Aceton, Acetonitril, Dimethylacetamid, DMSO bei Temperaturen über oder unter Raumtemperatur, zum Beispiel zwischen -80 °C bis 100 °C, vorzugsweise bei Raumtemperatur, vorgenommen werden.

Die Oxidation der 1-Hydroxygruppe wird nach den dem Fachmann bekannte Methoden vorgenommen. Als Oxidationsmittel können beispielsweise dienen: Pyridiniumdichromat (Tetrahedron Letters, 1979, 399), Jones-Reagenz (J. Chem. Soc. 1953, 2555) oder Platin/Sauerstoff (Adv. in Carbohydrate Chem. 17, 169 (1962) oder Collins-Oxidation und anschließende Jones-Oxidation. Die Oxidation mit Pyridiniumdichromat wird bei Temperaturen von 0 °C bis 100 °C, vorzugsweise 20 °C bis 40 °C in einem gegen das Oxidationsmittel inerten Lösungsmittel, beispielsweise Dimethylformamid, durchgeführt.

Die Oxidation mit Jones-Reagenz wird bei Temperaturen von -40 °C bis +40 °C, vorzugsweise 0 °C bis 30 °C in Aceton als Lösungsmittel ausgeführt.

Die Oxidation mit Platin/Sauerstoff wird bei Temperaturen von 0 °C bis 60 °C, vorzugsweise 20 °C bis 40 °C in einem gegen das Oxidationsmittel inerten Lösungsmittel, wie z. B. Essigester, durchgeführt.

Die Verseifung der Ester der Formel I wird nach den dem Fachmann bekannten Methoden durchgeführt, wie beispielsweise mit basischen Katalysatoren. Die Verbindungen der Formel I können durch die üblichen Trennmethoden in die optischen Isomeren getrennt werden.

Die Freisetzung der funktionell abgewandelten Hydroxygruppen erfolgt nach bekannten Methoden. Beispielweise wird die Abspaltung von Hydroxyschutzgruppen, wie beispielsweise des Tetrahydropyranylrestes, in einer wässrigen Lösung einer organischen Säure, wie z.B. Oxalsäure, Essigsäure, Propionsäure u.a., oder in einer wässrigen Lösung einer anorganischen Säure, wie z. B. Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind z. B. Alkohole, wie Methanol und Ethanol, und Ether, wie Dimethoxyethan, Dioxan und Tetrahydrofuran. Tetrahydrofuran wird bevorzugt angewendet. Die Abspaltung wird vorzugsweise bei Temperaturen zwischen 20 °C und 80 °C durchgeführt. Die Abspaltung der Silyletherschutzgruppen erfolgt beispielsweise mit Tetrabutylammoniumfluorid oder mit Kaliumfluorid in Gegenwart eines Kronenethers. Als Lösungsmittel sind beispielsweise geeignet Tetrahydrofuran, Diethylether, Dioxan, Methylenchlorid usw. Die Abspaltung wird vorzugsweise bei Temperaturen zwischen 0 °C und 80 °C durchgeführt.

Die Verseifung der Acylgruppen erfolgt beispielsweise mit Alkali- oder Erdalkali-carbonaten oder -hydroxyden in einem Alkohol oder in der wässrigen Lösung eines Alkohols. Als Alkohol kommen aliphatische Alkohole in Betracht, wie z.B. Methanol, Ethanol, Butanol usw., vorzugsweise Methanol. Als Alkalicarbonate und -hydroxyde seien Kalium- und Natriumsalze genannt. Bevorzugt sind die Kaliumsalze.

Als Erdkalicarbonate und -hydroxide sind beispielsweise geeignet Calciumcarbonat, Calciumhydroxyd und Bariumcarbonat. Die Umsetzung erfolgt bei -10 °C bis +70 °C, vorzugsweise bei +25 °C.

Die Einführung der Estergruppe 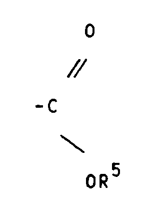
für R¹, bei welcher R⁵ eine Alkylgruppe mit 1-10 C-Atomen darstellt, erfolgt nach den dem Fachmann bekannten Methoden. Die 1-Carboxy-verbindungen werden beispielsweise mit Diazokohlenwasserstoffen in an sich bekannter Weise umgesetzt. Die Veresterung mit Diazokohlenwasserstoffen erfolgt z. B. dadurch, daß man eine Lösung des Diazokohlenwasserstoffes in einem inerten Lösungsmittel, vorzugsweise in Diethylether, mit der 1-Carboxyverbindung in dem gleichen oder in einem anderen inerten Lösungsmittel, wie z. B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seiten 389 - 394 (1954)].

Die Einführung der Estergruppe 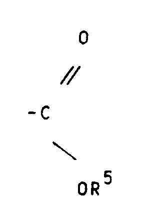
für R¹, bei welcher R⁵ eine substituierte oder unsubstituierte Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base, beispielsweise Pyridin, DMAP, Triethylamin, in einem inerten Lösungsmittel umgesetzt. Als Lösungsmittel kommen Methylenchlorid, Ethylenchlorid, Chloroform, Essigester, Tetrahydrofuran, vorzugsweise Chloroform in Frage. Die Reaktion wird bei Temperaturen zwischen -30 °C und +50 °C, vorzugsweise bei 10 °C, durchgeführt.

Sollen im Primärprodukt enthaltene C=C-Doppelbindungen reduziert werden, erfolgt die Hydrierung nach an sich bekannten Methoden.

Die Hydrierung des Δ^{8,10}-Diensystems wird in an sich bekannter Weise bei tiefen Temperaturen, vorzugsweise bei etwa -20 °C bis +30 °C in einer Wasserstoffatmosphäre in Gegenwart eines Edelmetallkatalysators durchgeführt. Als Katalysator ist zum Beispiel 10 % Palladium auf Kohle geeignet.

Die Leukotrien-B₄-Derivate der Formel I mit R⁵ in der Bedeutung eines Wasserstoffs können mit geeigneten Mengen der entsprechenden anorganischen Basen unter Neutralisierung in ein Salz überführt werden. Beispielsweise erhält man beim Lösen der entsprechenden Säuren in Wasser, das die stöchiometrische Menge der Base enthält, nach Abdampfen des Wassers oder nach Zugabe eines mit Wasser mischbaren Lösungsmittels, z.B. Alkohol oder Aceton, das feste anorganische Salz.

Zur Herstellung eines Aminsalzes wird die LTB₄-Säure z.B. in einem geeigneten Lösungsmittel, beispielsweise Ethanol, Aceton, Diethylether, Acetonitril oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die Einführung der Amidgruppe 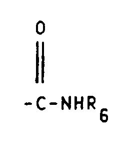
mit R₆ in der Bedeutung von Alkanoyl erfolgt nach den dem Fachmann bekannten Methoden. Die Carbonsäuren der Formel I (R₅=H), werden zunächst in Gegenwart eines tertiären Amins, wie beispielsweise Triethylamin, mit Chlorameisensäureisobutylester in das gemischte Anhydrid überführt. Die Umsetzung des gemischten Anhydrids mit dem Alkalisalz des entsprechenden Amids oder mit Ammoniak (R₆=H) erfolgt in einem inerten Lösungsmittel oder Lösungsmittelgemisch, wie beispielsweise Tetrahydrofuran, Dimethoxyethan, Dimethylformamid, Hexamethylphosphorsäuretriamid, bei Temperaturen zwischen -30 °C und +60 °C, vorzugsweise bei 0 °C bis 30 °C.

Eine weitere Möglichkeit für die Einführung der Amidgruppe 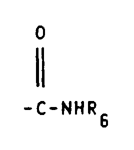
besteht in der Umsetzung einer 1-Carbonsäure der Formel I (R₅=H), in der freie Hydroxygruppen gegebenenfalls intermediär geschützt sind, mit Verbindungen der Formel IV,

O = C = N - R₆ (IV),

worin R₆ die oben angegebene Bedeutung hat.

Die Umsetztung der Verbindung der Formel I (R₅=H) mit einem Isocyanat der Formel IV erfolgt gegebenenfalls unter Zusatz eines tertiären Amins, wie z.B. Triethylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diethylether, Toluol, bei Temperaturen zwischen -80 °C bis 100 °C, vorzugsweise bei 0 °C bis 30 °C, vorgenommen werden.

Zur Herstellung der übrigen Amide kann man beispielsweise die gewünschten Säureanhydride mit Ammoniak oder den entsprechenden Aminen umsetzen.

Enthält das Ausgangsprodukt OH-Gruppen im Leukotrien-B₄-Rest so werden diese OH-Gruppen auch zur Raktion gebracht. Werden letztlich Endprodukte gewünscht, die freie Hydroxylgruppen enthalten, geht man zweckmässigerweise von Ausgangsprodukten aus, in denen diese durch vorzugsweise leicht abspaltbare Ether- oder Acylreste intermediär geschützt sind.

Die Trennung der Enantiomeren und/oder Diastereomeren erfolgt nach den dem Fachmann bekannten Methoden, beispielsweise Hochdruckflüssigchromatographie an optisch aktiven Trägermaterialien.

Die als Ausgangsmaterial dienenden Verbindungen der Formel II können beispielsweise hergestellt werden, indem man in an sich bekannter Weise cis- oder trans-Bis-1,2-Hydroxymethyl-cyclopentan (durch Reduktion aus cis oder trans Cyclopentan-1,2-dicarbonsäure erhältlich, siehe z.B. A.Padwa et al, J.Org.Chem. 54,817(1989); O.Caamaus et al., Eur.J.Med.Chem.22,311 (1987)) in den Monosilylether der Formel V 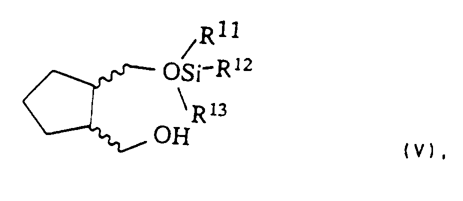
worin
R¹¹, R¹² und R¹⁵ gleich oder verschieden sind und C₁-C₄-Alkyl oder Phenyl bedeuten, überführt.

Durch Oxidation z.B. mit Collins-Reagenz oder durch das Swern-Verfahren wird der Aldehyd der Formel VI erhalten, 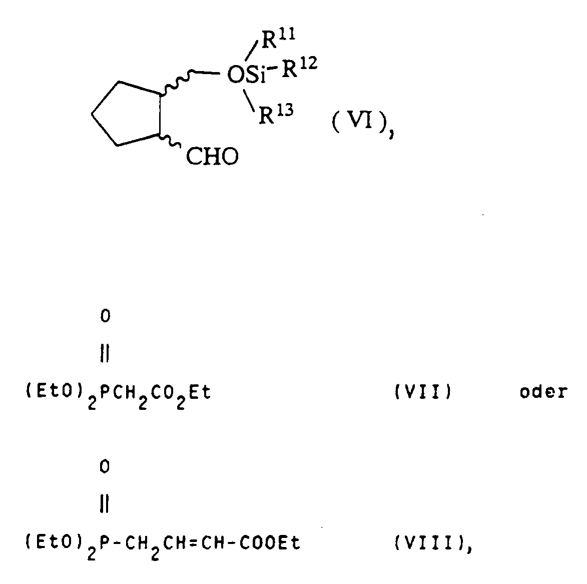
der in einer Wittig-Horner-Olefinierung mit dem Phosphonat der Formel VII und einer Base und gegebenenfalls anschließender Hydrierung sowie anschließender Reduktion der Estergruppe, Oxidation des primären Alkohols, nochmaliger Wittig-Horner-Olefinierung mit dem Phosphonat der Formel VII und gegebenenfalls anschließender Hydrierung in den Ester der Formel IX oder einer Wittig-Horner-Reaktion des Aldehyds der Formel VI mit einem Phosphat der Formel VIII überführt wird, wobei A die 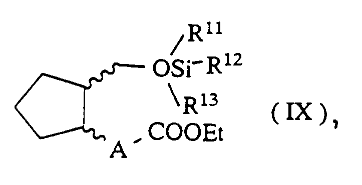
oben angegebene Bedeutung besitzt. Als Basen kommen beispielsweise Kaliumtert.-butylat, Diazabicyclononan oder Natriumhydrid infrage. Reduktion der Estergruppe, beispielsweise mit DIBAH und anschließende Oxidation des erhaltenen primären Alkohols z.B. mit Braunstein oder Collinsreagenz führt zum Aldehyd der Formel X 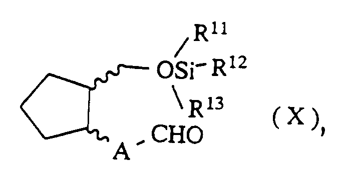
Die metallorganische Umsetzung des Aldehyds der Formel X mit einem Grignardreagenz der Formel XI, worin B, D

X-Mg-B-D-R₄ (XI),

und R⁴ die oben angegebenen Bedeutungen aufweisen und X Chlor, Brom oder Jod bedeutet, führt nach Schutz der Hydroxygruppe und gegebenenfalls Diastereomerentrennung (beispielsweise durch Acylierung) zu den Verbindungen der Formel XII 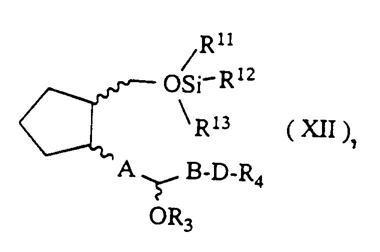
Die Herstellung der für die metallorganische Umsetzung benötigten Verbindung der Formel XI erfolgt durch Reaktion des entsprechenden terminalen Halogenids mit Magnesium. Durch Umsetzung des Silylethers XII mit Tetrabutylammoniumfluorid wird der Alkohol der Formel XIII erhalten. 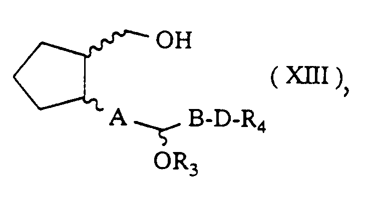
Die Oxidation der primären Alkoholgruppe in XIII z. B. Collinsreagenz oder Pyridiniumdichromat führt zum Aldehyd der Formel II.

Zu den Verbindungen der Formel XII, worin B eine CH₂-Gruppe und D eine -ClC- oder CH=CR⁸-Gruppe bedeutet, kann man gelangen, beispielsweise durch eine metallorganische Umsetzung eines Propargylhalogenids und anschließende Alkylierung mit einem entsprechenden Alkylhalogenid und gegebenenfalls anschließende Lindlar-Hydrierung.

Ein alternativer Aufbau der unteren Kette geht von dem Aldehyd der Formel XIV aus, der aus der Wittig-Horner-Umsetzung des Aldehyds VI und anschließender Reduktion und Oxidation resultierte. 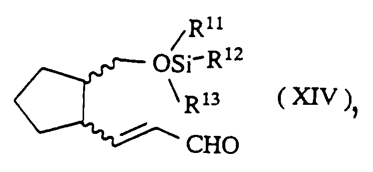
Wittig-Horner-Olefinierung des Aldehyds XIII mit einem Phosphonat der Formel XV 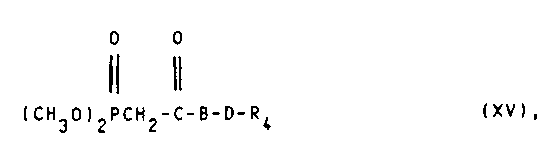
und Reduktion des entstandenen Ketons führte dann zum Alkohol der Formel XIII.

Der Einbau der chemisch und metabolisch labilen cis-Δ^{6,7}-Doppelbindung des LTB₄ in einen cis- oder trans-1,2-substituierten Cyclopentylring führt zu einer Stabilisierung, wobei insbesondere durch weitere Derivatisierung der funktionellen Gruppen, LTB₄-Derivate erhalten werden, die als LTB₄-Antagonisten wirken können.

Die Verbindungen der Formel I wirken antientzündlich und antiallergisch. Daneben besitzen sie antimykotische Eigenschaften. Folglich stellen die neuen Leukotrien B₄-Derivate der Formel I wertvolle pharmazeutische Wirkstoffe dar. Die Verbindungen der Formel I sind besonders zur topischen Applikation geeignet, da sie eine Dissoziation zwischen erwünschter topischer Wirksamkeit und unerwünschten systemischen Nebenwirkungen aufweisen.

Die neuen Leukotrien B₄-Derivate der Formel I eignen sich in Kombination mit den in der galenischen Pharmazie üblichen Hilfs- und Trägermitteln zur lokalen Behandlung von Kontaktdermatitis, Ekzemen der verschiedensten Art, Neurodermatosen, Erythrodermie, Pruritis vulvae et ani, Rosacea, Erythematodes cutaneus, Psoriasis, Lichen ruber planus et verrucosus und ähnlichen Hauterkrankungen.

Die Herstellung der Arzneimittelspezialitäten erfolgt in üblicher Weise, indem man die Wirkstoffe mit geeigeneten Zusätzen in die gewünschte Applikationsform, wie zum Beispiel: Lösungen, Lotionen, Salben, Cremes oder Pflaster, überführt,

In den so formulierten Arzneimitteln ist die Wirkstoffkonzentration von der Applikationsform abhängig. Bei Lotionen und Salben wird vorzugsweise eine Wirkstoffkonzentration von 0,0001 % bis 1 % verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Leukotrien-B₄-Derivate auch in Form von Kapseln, Tabletten oder Dragees, die vorzugsweise o,1 bis 100 mg Wirkstoff enthalten oder oral appliziert werden oder in Form von Suspensionen, die vorzugsweise 1-200 mg Wirkstoff pro Dosiseinheit enthalten und rektal appliziert werden auch zur Behandlung allergischer Erkrankungen des Darmtraktes, wie der Kolitis ulcerosa und der colitis granulomatosa.

Die neuen Leukotrien-B₄-Derivate können auch in Kombination, wie z.B mit Lipoxygenasehemmern, Cyclooxygenasehemmern, Prostacyclinagonisten, Thromboxanantagonisten, Leukotrien D₄-antagonisten, Leukotrien E₄-antagonisten, Leukotrien F₄-antagonisten, Phosphodiesterasehemmern, Calciumantagonisten oder PAF-Antagonisten verwendet werden.

Die nachfolgenden Ausführungsbeispiele dienen zur näheren Erläuterung des erfindungsgemäßen Verfahrens. In den Beispielen wurden nicht näher charakterisierte Diastereoisomere in der 12-Position als polar beziehungsweise unpolar charakterisiert (z. B. Diastereomer unpol (12)).

### Beispiele

### Beispiel 1

### (+/-)-(5RS)-5-Acetoxy-5-[cis-(2RS)-2-((1E,3E)-(5RS)-5-acetoxy-1,3-trideca-dienyl)(1RS)-cyclopentyl]-pentansäure Diastereomer unpol (12)

Zu 2,44 g Magnesium tropft man bei 25°C unter Argon eine Lösung aus 11,1g 4-Chlor-1-(tert.-butyldimethylsilyloxy)-butan in 10 ml Tetrahydrofuran, fügt einen Kristall Jod hinzu, erwärmt 10 Minuten auf 70°C, rührt 30 Minuten bei 25°C und verdünnt mit 31 ml Tetrahydrofuran.

Zu 2,96 ml dieser magnesiumorganischen Lösung tropft man bei -70°C unter Argon eine Lösung aus 495 mg cis-(1RS)-1-Formyl-(2RS)-2[(1E,3E)-(5RS)-5-acetoxy-1,3-tridecadienyl]-cyclopentan (Diastereomeres unpol (12)) in 1,7 ml Tetrahydrofuran und rührt 1,5 Stunden bei -70°C. Man versetzt mit 8 ml gesättigter Ammoniniumchloridlösung, extrahiert dreimal mit Ether, schüttelt die organische Phase mit Sole, trocknet über Natriumsulfat und dampft im Vakuum ein. Den Rückstand chromatographiert man an Kieselgel. Mit Hexan/Ether (8+2) erhält man 480 mg des Alkohols als farbloses Öl.
- IR(CHCl₃):: 3600,2930,2860,1725,1373,1450,993,836 cm⁻¹.

Zur Acetylierung fügt man zu einer Lösung aus 1,8 g des vorstehend beschriebenen Alkohols in 5 ml Pyridin 2,0 ml Esssigsäureanhydrid und rührt 23 Stunden bei Raumtemperatur. Anschließend engt man im Vakuum unter Zusatz von Toluol ein und chromatographiert den Rückstand an Kieselgel. Mit Hexan/Ether (8+2) erhält man 1,88 g des Acetats als farbloses Öl.
- IR:: 2930, 2862, 1730, 1610, 1375, 1255, 993, 840 cm⁻¹.

Zur Silylätherspaltung rührt man 1,35 g des vorstehend hergestellten Acetats in 80 ml Tetrahydrofuran mit 2,26 g Tetrabutylammoniumfluorid 20 Minuten bei 0°C und 4 Stunden bei 24°C unter Argon. Anschließend verdünnt man mit Ether, wäscht dreimal mit Wasser, trocknet über Magnesiumsulfat und dampft im Vakuum ein. Den Rückstand chromatographiert man mit Ether an Kieselgel. Dabei erhält man 960 mg des 1-Alkohols als farbloses Öl.
- IR:: 3600(breit), 2930. 2860, 1736, 1610, 1373, 1250, 990 cm⁻¹.

Zur Oxidation der 1-Hydroxygruppe fügt man zu 940 mg des vorstehend hergestellten Alkohols in 54 ml Methylenchlorid bei 0°C 5,4 g Collins-Reagenz und rührt 20 Minuten bei 0°C. Anschließend verdünnt man mit einer Mischung aus Hexan/Ether (1+1), fügt Celite hinzu, filtriert, wäscht mit Hexan/Ether (1+1) und dampft im Vakuum ein. Der so erhaltene 1-Aldehyd wird sofort ohne weitere Reinigung verwendet.

Zu einer Lösung von 800 mg des vorstehend hergestellten Aldehyds in 29 ml Aceton tropft man unter Rühren bei -25°C 1,67 ml Jones-Reagenz (J.Chem. Soc. 1953, 2555) und rührt 15 Minuten bei -25°C unter Argon. Anschließend fügt man 5,8 ml Isopropanol zu, rührt 5 Minuten, verdünnt mit 200 ml Ether, schüttelt zweimal mit Sole, trocknet über Magnesiumsulfat und dampft im Vakuum ein. Den Rückstand chromatographiert man an Kieselgel. Mit Hexan/Ether (1+1) erhält man 570 ml der Titelverbindung als farbloses Öl.
- IR:: 3520 (breit), 2930, 2860, 1726, 1373, 1255, 990, 948 cm⁻¹.

Das Ausgangsmaterial für die obige Titelverbindung wird wie folgt hergestellt:
1a) 3-[cis-1-(tert.-Butyl-dimethylsilyloxymethyl)-cyclopent-2-yl]-(2E)-propensäureäthylester
   Zu einer Lösung aus 8,7 g 2-Hydroxymethyl-cyclopentancarbonsäurelacton (O.Caamano et al., Euro.J.Med.Chem.22, 311(1987)) in 127 ml Toluol tropft man bei 0°C unter Argon 127 ml einer 1,2 molaren Lösung von Diisobutylaluminiumhydrid in Toluol und rührt 50 Minuten bei 0°C. Anschlißend tropft man 25 ml Isopropanol und 63 ml Wasser zu, rührt 2 Stunden bei 22°C, filtriert, wäscht mit Methylenchlorid und dampft im Vakuum ein. Den Rückstand reinigt man durch Chromatographie an Kieselgel. Mit Essigester/Hexan (4+1) erhält man 7g cis-1,2-Dihydroxymethyl-cyclopentan als farblose Flüssigkeit.
   - IR:: 3600, 3400, 2960, 1060 cm⁻¹.

   Zu einer Suspension von 4,36 g Natriumhydrid (als 55%ige Suspension in Mineralöl) in 200 ml Tetrahydrofuran tropft man bei 22°C eine Lösung von 13 g des vorstehend hergestellten Diols in 5 ml Tetrahydrofuran und rührt 45 Minuten bei 22°C. Man fügt anschließend 15 g tert.-Butyldimethylsilylchlorid zu, rührt 45 Minuten bei 22°C und verdünnt dann mit ca. 1,5 Liter Ether. Man wäscht den Etherextrakt mit 10%iger Kaliumcarbonatlösung, schüttelt dreimal mit Wasser, trocknet mit Natriumsulfat und dampft im Vakuum ein. Den Rückstand chromatographiert man an Kieselgel. Mit Hexan/Ether (95+5) erhält man 20,9 cis-1-(tert.-butyl-dimethylsilyloxymethyl)-2-hydroxymethylcyclopentan als farblose Flüssigkeit.
   - IR:: 3420 (breit), 2960, 2863, 1260, 840 cm⁻¹.

   Zu einer Lösung von 16,9 g des vorstehend beschriebenen Monosilylethers in 835 ml Methylenchlorid fügt man bei 0°C 84 g Collins-Reagenz (Chromsäure-Pyridin-Komplex) und rührt 30 Minuten bie 0°C. Anschließend verdünnt man mit einer Mischung aus Hexan/Ether (3+2), fügt Celite hinzu, filtriert und dampft im Vakuum ein. Man erhält 16,2 g des Aldehyds, der ohne weitere Reinigung verwendet wird.
   - IR:: 2958, 2930, 2860, 2740, 1713, 840 cm⁻¹.

   Zur Wittig-Olefinierung fügt man bei 24°C 20,7 g Phosphonoessigsäuretriäthylester und 12,6 g Diazabicycloundecen (DBU) zu einer gerührten Suspension von 3,9 g Lithiumchlorid in 277 ml Acetonitril und rührt 15 Minuten. Anschließend tropft man eine Lösung von 16 g des vorstehend beschriebenen Aldehyds in 43 ml Acetonitril zu, rührt 2,5 Stunden bei 24°C und verdünnt dann mit Ether. Man schüttelt nacheinander mit Wasser, 10%iger Schwefelsäure und Wasser, trocknet mit Natriumsulfat und dampft im Vakuum ein. Den Rückstand chromatographiert man mit Hexan/Ether (95+5) an Kieselgel. Dabei erhält man 22,5 g der Titelverbindung als farbloses Öl.
   - IR:: 2960, 2860, 1710, 1650, 1260, 985, 840 cm⁻¹.
1b) 5-[cis-1-(tert.-Butyl-dimethylsilyloxymethyl)-cyclo-pent-2-yl]-(2E,4E)pentadiensäureäthylester
   Zu einer Lösung von 22,5 g des nach Beispiel 1a hergestellten α,β-ungesättigten Esters in 500 ml Toluol tropft man bei -70°C unter Argon 120 ml einer 1,2 molaren Lösung von Diisobutylaluminiumhydrid in Toluol und rührt 30 Minuten bei -70°C. Anschließend tropft man 30 ml Isopropanol und dann 60 ml Wasser zu, rührt 2 Stunden bei 22°C, filtriert, wäscht mit Methylenchlorid und dampft im Vakuum ein. Man erhält 22,5 g des Allylalkohols, der ohne weitere Reinigung verwendet wird.
   - IR:: 3600, 3400, 2958, 840 cm⁻¹.

   Eine Lösung von 20,35 g des vorstehend hergestellten Alkohols in 654 ml Toluol versetzt man mit 80 g Braunstein und rührt 5 Stunden bei 24°C. Anschließend wird filtriert, eingedampft und an Kieselgel chromatographiert. Mit Hexan/Ether (92+8) eluiert man 17,6 g des Aldehyds als farbloses Öl.
   - IR:: 2960, 2860, 2745, 1730, 1633, 1470, 975, 840 cm⁻¹.

   Zur Wittig-Olefinierung fügt man bei 24° C 20,5 g Phosphonoessigsäuretriethylester und 12,4 g Diazabicycloundecen zu einer gerührten Suspension von 3,88 g Lithiumchlorid in 274 ml Acetonitril und rührt 15 Minuten. Anschließend tropft man eine Lösung von 17,5 g des vorstehend beschriebenen α,β-ungesättigten Aldehyds in 43 ml Acetonitril zu, rührt 4 Stunden bei 24° C und verdünnt dann mit Ether. Man schüttelt nacheinander mit Wasser, 10 %iger Zitronensäurelösung und Wasser, trocknet mit Natriumsulfat und dampft im Vakuum ein. Den Rückstand chromatographiert man mit Hexan/Ether (9+1) an Kieselgel. Dabei erhält man 15,7 g der Titelverbindung als farbloses Öl.
   - IR:: 2958, 2860, 1705, 1640, 1616, 1255, 1003, 970, 838 cm⁻¹.
1c) 5-[cis-1-(tert.-Butyl-dimethylsilyloxy-methyl-cyclo-pent-2-yl]-pentadien-1-ol
   Zu einer Lösung von 15 g des nach Beispiel 1b hergestellten Esters in 416 ml Toluol tropft man bei -70°C unter Argon 81 ml einer 1,2 molaren Lösung von Diisobutylaluminiumhydrid in Toluol und rührt 30 Minuten bei -70°C. Anschließend tropft man 15 ml Isopropanol und dann 40 ml Wasser zu, rührt 3 Stunden bei 23°C. filtriert, wäscht mit Methylenchlorid und dampft im Vakuum ein. Den Rückstand reinigt man durch Chromatographie an Kieselgel. Mit Hexan/Ether (8+2) erhält man 9,3 g des Alkohols als farbloses Öl.
   - IR:: 3620, 3460, 838 cm⁻¹.

   Eine Lösung von 9,3 g des vorstehend hergestellten Alkohols in 273 ml Toluol versetzt man mit 27,3 g Braunstein und rührt 6 Stunden bei 24°C. Anschließend wird filtriert, eingedampft und an Kieselgel chromatographiert. Mit Hexan/Ether (9+1) erhält man 6,9 g der Titelverbindung als farbloses Öl.
   - IR:: 2955, 2858, 2740, 1678, 1632, 986, 838 cm⁻¹.
1d) (5RS)-5-Acetoxy-1-[cis-1-(tert.-butyl-dimethylsilyloxymethyl)-cyclopent)-2-yl]-(1E,3E)-tridecadien
   Zu 2,24 g Magnesium in 12 ml Ether tropft man unter Erwärmen eine Lösung aus 17,8 g Octylbromid in 24 ml Ether und rührt 30 Minuten bei 25°C. Zu 17,2 ml (31,7 mMol) dieser Grignardlösung tropft man bei -20°C unter Argon eine Lösung aus s6,7 g des nach Beispiel 1c hergestellten Aldehyds in 100 ml Ether und rührt 45 Minuten bei -20°C. Man versetzt mit gesättigter Ammoniumchloridlösung, extrahiert dreimal mit Ether, schüttelt die organische Phase mit Sole, trocknet über Natriumsulfat und dampft im Vakuum ein. Den Rückstand chromatographiert man an Kieselgel. Mit Hexan/Essigestergemischen erhält man 3,35 des unpolaren diastereomeren Alkohols und 3,6 g des polaren diastereomeren Alkohols als farblose Öle.
   Zur Acetylierung fügt man zu einer Lösung von 3,35 g des vorstehend hergestellten unpolaren diastereomeren Alkohols in 8 ml Pyridin 3,1 ml Essigsäureanhydrid und rührt 24 Stunden bei Raumtemperatur.
   Anschließend engt man im Vakuum unter Zusatz von Toluol ein und chromatographiert den Rückstand an Kieselgel. Mit Hexan/Ether (95+5) erhält man 3,5 g der Titelverbindung (unpolares Diastereomares) als farbloses Öl.
   - IR:: 2938, 2860, 1725, 1655, 1252, 990, 838 cm⁻¹.

   In analoger Weise wird aus 3,6 g des vorstehend hergestellten polaren diastereomeren Alkohols mit 3,3 ml Essigsäureanhydrid und 8,6 ml Pyridin 3,7 g der Titelverbindung (als polares Diastereomeres) hergestellt.
   - IR:: 2915, 2860, 1725, 1655, 1252, 990, 838 cm⁻¹.
1e) cis-(1RS)-1-Formyl-(2RS)-2-[(1E,3E)-(5RS)-5-acetoxy-1,3-tridecadienyl]cyclopentan Diastereomer unpol(12)
   Zu einer Lösung von 3,5 g des nach Beispiel 1d hergestellten Acetates (unpolares Diastereomer) in 350 ml Tetrahydrofuran fügt man bei 0°C 7,35 g Tetrabutylammoniumfluorid, rührt 15 Minuten bei 0°C und 5,5 Stunden bei 24°C. Anschließend verdünnt man mit 1 Liter Ether und wäscht dreimal mit Sole. Man trocknet über Magnesiumsulfat, dampft im Vakuum ein und chromatographiert den Rückstand an Kieselgel. Mit Hexan/Ether (7+3) eluiert man 2,45 g des Alkohols als farbloses Öl.
   - IR:: 3620, 3450, 2930, 2860, 1725, 1260, 992 cm⁻¹.

   Zu einer Lösung von 2,4 g des vorstehend hergestellten Alkohols in 75 ml Methylenchlorid fügt man bei 0°C 18,4 g Collins-Reagenz (Chromsäure-Pyridin-Komplex) und rührt 20 Minuten bei 0°C. Anschließend verdünnt man mit einer Mischung aus Hexan/Ather (1+1), fügt Celite hinzu, filtriert und dampft im Vakuum ein. Der so erhaltene Aldehyd wurde ohne weitere Reinigung verwendet.
   - IR:: 2930, 2860, 2730, 1721, 1250, 990 cm⁻¹

### Beispiel 2

### (+/-)-(5RS)-5-Hydroxy-5-[cis-(2RS)-2-((1E,3E)-(5RS)-5-hydroxy-1,3-tridecadienyl)-(1RS)-cycopentyl]-pentansäure Diastereomer unpol (12)

Zu einer Lösung von 360 mg des nach Beispiel 1 hergestellten Diacetats in 7,7 ml Methanol fügt man 7,7 ml einer 0,5 normalen wäßrigen Lithiumhydroxidlösung und rührt 25 Stunden bei 50°C. Anbschließend säuert man mit einer 10%igen Schwefelsäure auf pH 5 an, verdünnt mit Essigester, schüttelt dreimal mit Sole, trocknet mit Natriumsufat und dampft im Vakuum ein. Den Rückstand chromatographiert man mit Ather/Athanol (99+1) an Kieselgel. Dabei erhält man 197 mg der Titelverbindung als farblose Kristalle (Schmp. 71°C)
- IR:: 3400, 2928, 2850, 1725, 1360, 1230, 995, 930 cm⁻¹.

### Beispiel 3

### (+/-)-(5RS)-5-Acetoxy-5-[cis-(2RS)-2-((1E,3E)-(5RS)-5-hydroxy-1,3-tridecadienyl)-(1RS)-cyclopentyl]-pentansäure Diastereomer unpol (12)

Zu einer Lösung von 255 mg des nach Beispiel 1 hergestellten unpolaren diastereomeren Diacetats in 5,5 ml Methanol fügt man bei 23°C 5,5 ml einer 0,5 normalen Natronlauge und rührt 1,5 Stunden bei 23°C unter Argon. Anschließend verdünnt man mit Wasser und säuert bei Eisbadtemperatur mit 10%iger Schwefelsäure auf pH 5 an. Man extrahiert mit Essigester, wäscht zweimal mit Sole, trocknet mit Natriumsulfat und dampft im Vakuum ein. Den Rückstand chromatographiert man an Kieselgel. Mit Ether/Hexan (8+2) erhält man 202 mg der Titelverbindung als farbloses Öl.
- IR:: 3420, 2922, 2850, 1727, 1710, 1700, 1272, 990, 960 cm⁻¹.

### Beispiel 4

### (+/-)-(5RS)-5-Hydroxy-5-[cis-(2RS)-2-((1E,3E)-(5RS)-5-hydroxy-1,3-tridecadienyl)-(1RS)-cyclopentyl]-pentan-1-ol Diastereomer unpol (12)

180 mg des in Beispiel 1 beschriebenen unpolaren diastereomeren Diacetats rührt man 60 Stunden bei 24°C mit 5,5 ml einer Lösung von Kaliumhydroxid in Wasser und Ethanol (Herstellung: man löst 5 g Kaliumhydroxid in 67,5 ml Wasser und 182,5 ml Ethanol). Anschließend säuert man mit 10%iger Zitronensäurelösung auf pH 6 an, extrahiert viermal mit je 20 ml Methylenchlorid,, schüttelt die organische Phase mit Sole, trocknet über Magnesiumsulfat und dampft im Vakuum ein. Den Rückstand chromatographiert man mit Essigester an Kieselgel. Dabei erhält man 103 mg der Titelverbindung als farbloses Öl.
- IR:: 3610, 3370 (breit), 2930, 2860, 993 cm⁻¹.

### Beispiel 5

### (+/-)-(5RS)-5-Acetoxy-5-[cis-(2RS)-2-((1E,3E)-(5RS)-5-acetoxy-1,3-tridecadienyl)-(1RS)-cyclopentyl]-pentansäure Diastereomer pol (12)

Zu 2,44 g Magnesium tropft man bei 25°C unter Argon eine Lösung aus 11,1 g 4-Chlor-1-(tert.-butyldimethnylsilyloxy)-butan in 10 ml Tetrahydrofuran, fügt einen Kristall Jod hinzu, erwärmt 10 Minuten auf 75°C, rührt 40 Minuten bei 25°C und verdünnt mit 31 ml Tetrahydrofuran.

Zu 9,6 ml dieser magnesiumorganischen Lösung tropft man bei -70°C unter Argon eine Lösung aus 1,6 g cis-(1RS)-1-Formyl-(2RS)-2-[(1E,3E)-(5RS)-5-acetoxy-1,3-tridecadienyl]-cyclopentan (Diastereomeres pol(12) in 5,5 ml Tetrahydrofuran und rührt 1 Stunde bei -70°C.

Man versetzt mit 30 ml gesättigter Ammoniumchloridlösung, extrahiert dreimal mit Ether, schüttelt die organische Phase mit Sole, trocknet über Natriumsulfat und dampft im Vakuum ein. Den Rückstand chromatographiert man an Kieselgel. Mit Hexan/Ether (8+2) erhält man 1,3 g des Alkohols als farbloses Öl.
- IR:: 3610, 2930, 2860, 1725, 1374, 1451, 993, 836 cm⁻¹.

Zur Acetylierung fügt man zu einer Lösung aus 1,82 g des vorstehend beschriebenen Alkohols in 5,1 ml Pyridin 2,1 g Essigsäureanhydrid und rührt 22 Stunden bei Raumtemperatur. Anschließend engt man im Vakuum unter Zusatz von Toluol ein und chromatographiert den Rückstand an Kieselgel. Mit Hexan/Ether (9+1) erhält man 1,83 g des Acetats als farbloses Öl.
- IR:: 2930, 2860, 1728, 1608, 1375, 1255, 993, 840 cm⁻¹.

Zur Silyletherspaltung rührt man 1,85 g des vorstehend hergestellten Acetats in 113 ml Tetrahydrofuran mit 3,1 g Tetrabutylammoniumfluorid 20 Minuten bei 0° C und 4 Stunden bei 24° C unter Argon. Anschließend verdünnt man mit Ether, wäscht dreimal mit Wasser, trocknet über Magnesiumsulfat und dampft im Vakuum ein. Den Rückstand chromatographiert man mit Ether/Hexan (8+2) an Kieselgel. Dabei erhält man 1,3 g des 1-Alkohols als farbloses Öl.
- IR:: 3620, 3500, 2930, 2860, 1725, 1608, 1375, 1250, 990 cm⁻¹.

Zur Oxidation der 1-Hydroxy-Gruppe fügt man zu 1,25 g des vorstehend hergestellten Alkohols in 71 ml Methylenchlorid 7,2 g Collins-Reagenz und rührt 20 Minuten bei 0° C. Anschließend verdünnt man mit einer Mischung aus Hexan/Ether (1+1), fügt Celite hinzu filtriert, wäscht mit Hexan/Ether (1+1) und dampoft im Vakuum ein. Der so erhaltene 1-Aldehyd wird sofort ohne weitere Reinigung verwendet.

Zu einer Lösung von 1,1 g des vorstehend hergestellten Aldehyds in 40 ml Aceton tropft man unter Rühren bei -25° C 2,3 ml Jones-REagenz (j.Chem. Soc. 1953, 2555) und rührt 15 Minuten bei -25° C unter Argon. Anschließend fügt man 8 ml Isopropanol zu, rührt 5 Minuten, verdünnt mit 300 ml Ether, schüttelt zweimal mit Sole, trocknet über Natriumsulfat und dampft im Vakuum ein. Den Rückstand chromatographiert man an Kieselgel. Mit Ether/Hexan (7+3) erhält man 681 mg der Titelverbindung als farbloses Öl.
- IR:: 3520 (breit),2930, 2859, 1725, 1373, 1250, 990, 948 cm⁻¹.

Das Ausgangsmaterial für die obige Titelverbindung wird wie folgt hergestellt:
5a) cis-(1RS)-1-Formyl-(2RS)-2-[(1E,3E)-(5RS)-5-acetoxy-1,3-tridecadienyl]-cyclopentan Diastereomer pol(12)
   Zu einer Lösung von 3,68 g des nach Beispiel 1 d hergestellten (5RS)-5-Acetoxy-1-[cis-1-(tert.-butyl-dimethylsilyloxymethyl)-cyclopent-2-yl]-(1E,3E)-tridecadiens (polares Diastereomer) in 360 ml Tetrahydrofuran fügt man bei 0° C 7,73 g Tetrabutylammoniumfluorid, rührt 30 Minuten bei 0° C und 5 Stunden bei 24° C. Anschließend verdünnt man mit 1 Liter Ether und wäscht dreimal mit Sole. Man trocknet über Magnesiumsulfat, dampft im Vakuum ein und chromatographiert den Rückstand an Kieselgel. Mit Hexan/Ether (7+3) eluiert man 2,3 g des Alkohols als farbloses Öl.
   - IR:: 3620, 3450, 2930, 2860, 1725, 1250, 991 cm⁻¹.

   Zu einer Lösung von 1,8 g des vorstehend hergestellten Alkohols in 56 ml Methylenchlorid fügt man bei 0° C 14 g Collins-Reagenz (Chromsäure-Pyridin-Komplex) und rührt 20 Minuten bei 0° C. Anschließend verdünnt man mit einer Mischung aus Hexan/Ether (1+1), fügt Celite hinzu, filtriert und dampft im Vakuum ein. Der so erhaltene Aldehyd wurde ohne weitere Reinigung verwendet.
   - IR:: 2930, 2860, 1720, 1250, 991 cm⁻¹.

### Beispiel 6

### (+/-)-(5RS)-5-Hydroxy-5-[cis-(2RS)-2-((1E,3E)-(5RS)-5-hydroxy-1,3-trideca-dienyl)-(1RS)-cyclopentyl]-pentansäure Diastereomer pol (12)

In Analogie zu Beispiel 2 erhalt man aus 360 mg des nach Beispiel 5 hergestellten Diacetats 174 mg der Titelverbindung als farbloses Öl.
- IR:: 3400, 2930, 2855, 1723, 1360, 1230, 995, 930 cm⁻¹.

### Beispiel 7

### (+/-)-(5RS)-5-Acetoxy-5-[cis-(2RS)-2-((1E,3E)-(5RS)-5-hydroxy-1,3-trideca-dienyl)-(1RS)-cyclopentyl]-pentansäure Diastereomer pol (12)

In Analogie zu Beispiel 3 erhält man aus 305 mg des nach Beispiel 5 hergestellten Diacetats 238 mg der Titelverbindung als farbloses Öl.
- IR:: 3520, 2930, 2860, 1723, 1250, 990, 962 cm⁻¹.

### Beispiel 8

### (+/-)-(5RS)-5-Hydroxy-5-[cis-(2RS)-2-((1E,3E)-(5RS)-5-hydroxy-1,3-trideca-dienyl)-(1RS)-cyclopentyl]-pentan-1-ol Diastereomer pol (12)

In Analogie zu Beispiel 4 erhält man aus 190 mg des nach Beispiel 5 hergestellten Diacetats (Zwischenprodukt mit der Hydroxygruppe in der 1-Position) 115 mg der Titelverbindung als farbloses Öl.
- IR:: 3600, 3380 (breit), 2930, 2860, 992 cm⁻¹.

### Beispiel 9

### (+/-)-(5RS)-5-Acetoxy-5-[trans-(2RS)-2-((1E,3E)-(5RS)-5-acetoxy-1,3-tridecadienyl)-1,3-tridecadienyl)-(1RS)-cyclopentyl]-pentansäure Diastereomer A

Zu 4,87 g Magnesium tropft man bei 25° C untert Argon eine Lösung aus 22,3 g 4-Chlor-1-(tert.-butyldimethylsilyloxy)-butan in 20 ml Tetrahydrofuran, fügt einen Kristall Iod hinzu, erwärmt 10 Minuten auf 70° C, rührt 30 Minuten bei 25° C und verdünnt mit 62,5 ml Tetrahydrofuran.

Zu 48,3 ml dieser magnesiumorganischen Lösung tropft man bei -70° C unter Argon eine Lösung aus 8,1 g trans-(1RS)-1-Formyl-(2RS)-2-[(1E,3E)-(5RS)-5-acetoxy-1,3-tridecadienyl]-cyclopentan in 27 ml Tetrahydrofuran und rührt 1,5 Stunden bei -70° C. Man versetzt mit 200 ml gesättigter Ammoniumchloridlösung, extrahiert mit Ether, schüttelt die organische Phase mit Sole, trocknet über Natriumsulfat und dampft im Vakuum ein. Den Rückstand chromatographiert man an Kieselgel mit Hexan/Essigester (85+15) erhält 8,2 g des Alkohols als farbloses Öl.
- IR:: 3450, 2930, 2860, 1725, 1374, 1255, 993, 840 cm⁻¹.

Zur Acetylierung fügt man zu einer Lösung aus 7,85 g des vorstehend hergestellten Alkohols in 50 ml Pyridin 8,6 ml Essigsäureanhydrid und rührt 21 Stunden bei Raumtemperatur. Anschließend engt man im Vakuum unter Zusatz von Toluol ein und chromatographiert den Rückstand an Kieselgel mit Hexan/Essigester (85+15) erhält man 7,6 g des Acetats als farbloses Öl.
- IR:: 2940, 2862, 1728, 1375, 1257, 992, 840 cm⁻¹.

Zur Silyletherspaltung rührt man 7,55 g des vorstehend hergestellten Acetats in 500 ml Tetrahydrofuran mit 12,7 g Tetrabutylammoniumfluorid 1 Stunde bei 0° und 4 Stunden bei 24° C unter Argon. Anschließend verdünnt man mit Ether, wäscht dreimal mit Wasser, trocknet über Magensiumsulfat und dampft im Vakuum ein. Den Rückstand chromatographiert man an Kieselgel. Mit Hexan/Essigestergemische erhält man durch mehrfache Chromatographien 1,7 g des unpolaren diastereomeren Alkohols (Diastereomer A) und 2,3 g des polaren diasteromeren Alkohols (Diastereomer B) als farbloses Öl.
- IR (unpolarer Alkohol):: 3630, 3500, 2938, 2862, 1727, 1660, 1378, 1255, 992, 950 cm⁻¹.
- IR (polarer Alkohol):: 3620, 3480, 2938, 2862, 1726, 1660, 1378, 1255, 992, 950 cm⁻¹.

Zur Oxidation der 1-Hydroxygruppe fügt man zu 1,55 g des vorstehend hergestellten unpolaren Alkohols (Diastereomer A) in 122 ml Methylenchlorid bei 0° C 10,7 g Collins-Reagenz und rührt 20 Minuten bei 0° C. Anschließend verdünnt man mit einer Mischung aus Hexan/Ether (1+1), fügt Celite hinzu, filtriert, wäscht mit Hexan/Ether (1+1) und dampft im Vakuum ein. Der so erhaltene 1-Aldehyd wird sofort ohne weitere Reinigung verwendet.

Zu einer Lösung von 1,57 g des vorstehend hergestellten Aldehyds in 58 ml Aceton tropft man unter Rühren bei -25° C 3,3 ml Jones-Reagenz (J. Chem. Soc. 1953, 2555) und rührt 15 Minuten bei -25° C unter Argon. Anschließend fügt man 11,5 ml Isopropanol zu, rührt 5 Minuten, verdünnt mit 400 ml Ether, schüttelt zweimal mit Sole, trocknet über Natriumsulfat und dampft im Vakuum ein. Den Rückstand chromatographiert man an Kieselgel. Mit Hexan/Essigester (6+4) erhält man 1,2 g der Titelverbindung als farbloses Öl.
- IR:: 3520, 2930, 2860, 1725, 1658, 1360, 1250, 990, 946 cm⁻¹.

Das Ausgangsmaterial für die obige Titelverbindung wird wie folgt hergestellt:

### 9a)

### 5-[trans-1-(tert.-Butyl-dimethylsilyloxymethyl)-cyclopent-2-yl]-2E,4E)-pentadiensäureethylester

In einer Lösung von 25 g trans-Cyclopentan-1,2-dicarbonsäure in 64 ml Methanol fügt man 3,4 ml konzentrierte Schwefelsäure und kocht 14 Stunden am Rückfluß. Anschließend destilliert man den überschüssigen Alkohol ab, gießt den Rückstand auf Eiswasser, extrahiert viermal mit Ether und wäscht die organische Phase mit Natriumbicarbonatlösung und Wasser. Man trocknet mit Natriumsulfat, engt im Vakuum ein und destilliert den Rückstand im Vakuum bei 25 mTorr. Bei 56 °C erhalt man 27 g trans-Cyclopentan-1,2-dicarbonsäuredimethylester als farblose Flüssigkeit.
- IR:: 2955, 2873, 1725, 1435 cm⁻¹

Zu einer Lösung aus 13 g trans-Cyclopentan-1,2-dicarbonsäuredimethylester in 300 ml Toluol tropft man bei 0 °C unter Argon 230 ml einer 1,2 molaren Lösung von Diisobutylaluminiumhydrid in Toluol und rührt 2 Stunden bei 0 ° C. Anschließend tropft min 20 ml Isopropanol zu, rührt 5 Minuten, tropft 116 ml Wasser zu und rührt 2 Stunden bei 22 °C. Man filtriert, wäscht mit Methylenchlorid und dampft im Vakuum ein. Den Rückstand reinigt man durch Chromatographie an Kieselgel. Mit Essigester erhält man 8 g trans-1,2-Dihydroxy-cyclopentan als farblose Flüssigkeit.
- IR:: 3610, 3400, 2960, 1062 cm⁻¹

Zu einer Lösung von 16 g trans-1,2-Dihydroxymethyl-cyclopentan in 146 ml Dimethylformamid fügt man bei 0 °C 17,1 g Imidazol und 18,9 g tert.-Butyldimethylsilylchlorid und rührt 22 Stunden bei 24 °C. Man verdünnt mit 1,6 l Ether, schüttelt zweimal mit je 80 ml 10%ige Schwefelsäure, wäscht mit Wasser neutral, trocknet über Magnesiumsulfat und dampft im Vakuum ein. Den Rückstand chromatographiert man an Kieselgel. Mit Hexan/Essigester (9+1) erhält man 12 g trans-1-(tert.-Butyl-dimethylsilyloxymethyl)-2-hydroxymethyl-cyclopentan als farblose Flüssigkeit.
- IR:: 3400, 2960, 2860, 1260, 840 cm ⁻¹
Zu einer Lösung von 14,4 g des vorstehend beschriebenen Monosilylethers in 750 ml Methylenchlorid fügt man 70 g Collins-Reagenz (Chromsäure-Pyridin-Komplex) und rührt 30 Minuten bei 0 °C. Anschließend verdünnt man mit einer Mischung aus Hexan/Ether (3+2), fügt Celite hinzu, filtriert und dampft im Vakuum ein. Man erhält 14 g des Aldehyds, der ohne weitere verwendet wird.
- IR:: 2958, 2860, 2720, 1719, 840 cm⁻¹
Zur Witig-Horner-Olefinierung fügt man bei 24 °C 16,1 g Phosphonocrotonsäuretriethylester und 9,8 g Diazabicycloundecen (DBU) zu einer gerührten Suspension von 2,7 g Lithiumchlorid in 536 ml Acetonitril und rührt 10 Minuten. Anschließend tropft man eine Lösung aus 13 g des vorstehend beschriebenen Aldehyds in 107 ml Acetonitril zu, rührt 3,5 Stunden bei 24 °C und verdünnt dann mit Ether. Man schüttelt nacheinander mit Wasser, 10%iger Zitronensäurelösung und Wasser, trocknet über Magnesiumsulfat und dampft im Vakuum ein. Den Rückstand chromatographiert man mit Hexan/Essigester an Kieselgel. Dabei erhält man 11 g der Titelverbindung als farbloses Öl.
- IR:: 2958, 2860, 1705, 1640, 1620, 1470, 1255, 1000, 838 cm⁻¹

### 9b)

### 5-[trans-1-(tert.-Butyl-dimethylsilyloxymethyl)-cyclopent-2-yl]-(2E,4E)-pentadien-1-al

Zu einer Lösung von 9,8 g des nach Beispiel 9a hergestellten Esters in 250 ml Toluol tropft man bei - 70 °C unter Argon 50 ml einer 1,2 molaren Lösung von Diisobutylaluminiumhydrid in Toluol und rührt 30 Minuten bei - 70 °C. Anschließend tropft man 10 ml Isopropanol und nach 5 Minuten 30 ml Wasser zu, rührt 2,5 Stunden bei 23 °C, filtriert, wäscht mit Methylenchlorid und dampft im Vakuum ein. Den Rückstand reinigt man durch Chromatographie an Kieselgel. Mit Hexan/Ether (7+3) erhält man 7,6 g des Alkohols als farbloses Öl.
- IR:: 3610, 3450, 990, 940, 840 cm⁻¹
Zur Aldehydherstellung versetzt man eine Lösung von 7,5 g des vorstehend hergestellten Alkohols in 250 ml Toluol mit 22 g Braunstein und rührt 4 Stunden bei 24 °C. Anschließend wird filtriert, eingedampft und an Kieselgel chromatographiert. Mit Hexan/Esigester (8+2) erhält man 7,4 g der Titelverbindung als farbloses Öl.
- IR:: 2960, 2860, 2740, 1682, 1638, 988, 940, 840 cm⁻¹

### 9c)

### (5-RS)-5-Acetoxy-1-[trans-1-(tert.-butyl-dimethylsilyloxymethyl)-cyclopent-2-yl]-1(1E,3E)-tridecadien

Zu 2,24 g Magnesium in 12 ml Ether tropft man unter Erwärmen eine Lösung aus 17,8 g Octylbromid in 24 ml Ether und rührt 30 Minuten bei 25 °C. Zu 16,4 ml (=30,1 mMol) dieser Grignardlösung tropft man bei - 20 °C unter Argon eine Lösung aus 7,4 g des nach Beispiel 9b hergestellten Aldehyds in 130 ml Ether und rührt 45 Minuten bei - 20 °C. Man versetzt mit gesättigter Ammoniumchloridlösung, extrahiert dreimal mit Ether, schüttelt die organische Phase mit Sole, trocknet über Natriumsulfat und dampft im Vakuum ein. Den Rückstand chromatographiert man an Kieselgel. Mit Hexan/Essigester (7+3) erhält man 8,6 g des Alkohols (Diastereomerengemisch) als farbloses Öl.

Zur Acetylierung fügt man zu einer Lösung von 8,5 g des vorstehend hergestellten Alkohols in 85 ml Pyridin 12,5 ml Essigsäureanhydrid und rührt 23 Stunden bei Raumtemperatur. Anschließend engt man im Vakuum unter Zusatz von Toluol ein und chromatographiert den Rückstand an Kieselgel. Mit Hexan/Essigester (97+3) erhält man 8,6 g der Titelverbindung als farbloses Öl.
- IR:: 2938, 2860, 1725, 1657, 1255, 993, 945, 840 cm⁻¹

### 9d)

### trans-(1RS)-1-Formyl-(2RS)-2-[(1E,3E)-(5RS)-5-acetoxy-1,3-tridecadienyl]-cylcopentan

Zu einer Lösung von 9,1 g des nach Beispiel 9c hergestellten Acetats in 900 ml Tetrahydrofuran fügt man bei 0 °C 19,9 g Tetrabutylammoniumfluorid, rührt 15 Minuten bei 0 °C und 4 Stunden bei 24 °C. Anschließend verdünnt man mit 2 l Ether und wäscht dreimal mit Sole. Man trocknet mit Natriumsulfat, dampft im Vakuum ein und chromatographiert den Rückstand an Kieselgel. Mit Hexan/Essigester (7+3) eluiert man 7,18 g des Alkohols als farbloses Öl.
- IR:: 3620, 3450, 2938, 2862, 1730, 1255, 995 cm⁻¹
Zu einer Lösung von 8,1 g des vorstehend hergestellten Alkohols in 250 ml Methylenchlorid fügt man bei 0 °C 63 g Collins-Reagenz (Chromsäure-Pyridin-Komplex) und rührt 20 Minuten bei 0 °C. Anschließend verdünnt man mit einer Mischung aus Hexan/Ether (1+1), fügt Celite hinzu, filtriert und dampft im Vakuum ein. Der so erhaltene Aldehyd wurde ohne weitere Reinigung verwendet.
- IR:: 2930, 2860, 2731, 1721, 1250, 990 cm⁻¹

### Beispiel 10

### (+/-)-(5RS)-5-Hydroxy-5-[trans-(2-RS)-2-(1E,3E)-(5RS)-5-hydroxy-1,3-tridecadienyl)-(1RS)-cyclopentyl]-pentansäure Diastereomer A

In Analogie zu Beispiel 2 erhält man aus 250 mg des nach Beispiel 9 hergestellten Diacetats 168 mg der Titelverbindung als farbloses Öl.
- IR (Film):: 3400, 2930, 2858, 1715, 1660, 1250, 989, 930 cm⁻¹

### Beispiel 11

### (+/-)-(5RS)-5-Acetoxy-5-[trans-(2RS)-2-(1E,3E)-(5RS)-5-hydroxy-1,3-tridecadienyl)-(1RS)-cyclopentyl]-pentansäure Diastereomer A

In Analogie zu Beispiel 3 erhalt man aus 250 mg des nach Beispiel 9 hergestellten Diacetats 204 mg der Titelverbindung als farbloses Ol.
- IR (Film):: 3450, 2930, 2860, 1736, 1713, 1660, 1245, 990 cm⁻¹

### Beispiel 12

### (+/-)-(5RS)-5-Acetoxy-5-[trans-(2RS)-2-(1E,3E)-(5RS)-5-acetoxy-1,3-tridecadienyl)-(1RS)-cyclopentyl]-pentansäure Diastereomer B

Zu einer Lösung von 2,2 g des in Beispiel 9 hergestellten polaren Alkohols (Diastereomer B) in 173 ml Methylenchlorid fügt man bei 0 °C 15 g Collinsreagenz und rührt 20 Minuten bei 0 °C. Anschließend verdünnt man mit einer Mischung aus Hexan/Ether (1+1), fügt Celite hinzu, filtriert, wäscht mit Hexan/Ether (1+1) und dampft im Vakuum ein. Der so erhaltene 1-Aldehyd wird sofort ohne weitere Reinigung verwendet.

Zu einer Lösung von 2,25 g des vorstehend hergestellten Aldehyds in 83 ml Aceton tropft man unter Rühren bei - 25 °C 4,7 ml Jones-Reagenz und rührt 12 Minuten bei - 25 °C unter Argon. Anschließend fügt man 16,5 ml Isopropanol zu, rührt 5 Minuten, verdünnt mit Ether, schüttelt mit Sole, trocknet über Natriumsulfat und dampft im Vakuum ein. Den Rückstand chromatographiert man an Kieselgel. Mit Hexan/Essigester (6+4) erhält man 1,6 g der Titelverbindung als farbloses Ol.
- IR:: 3620, 2930, 2860, 1721, 1658, 1370, 1250, 989, 946 cm⁻¹

### Beispiel 13

### (+/-)-(5RS)-5-Hydroxy-5-[trans-(2RS)-2-(1E,3E)-(5S)-5-hydroxy-1,3-tridecadienyl)-(1RS)-cyclopentyl]-pentansäure Diastereomer B

In Analogie zu Beispiel 2 erhält man aus 250 mg des nach Beispiel 12 hergestellten Diacetats 178 mg der Titelverbindung als farbloses Öl.
- IR:: 3600, 3420, 2930, 2859, 1730, 1660, 1250, 990 cm⁻¹

### Beispiel 14

### (+/-)-(5RS)-5-Acetoxy-5-[trans-(2RS)-2-(1E,3E)-(5S)-5-hydroxy-1,3-tridecadienyl)-(1RS)-cyclopentyl]-pentansäure Diastereomer B

In Analogie zu Beispiel 3 erhält man aus 250 mg des nach Beispiel 12 hergestellten Diacetats 187 mg der Titelverbindung als farbloses Öl.
- IR (Film):: 3450, 2925, 2850, 1730, 1708, 1660, 1245, 985 cm⁻¹

### Beispiel 15

### (+/-)-(5RS)-5-Hydroxy-5-[cis-(2RS)-2-(1E,3E)-(5S)-5-hydroxy-1,3-tridecadienyl)-(1RS)-cyclopentyl]-pentansäuremethylester Diastereomer unpol (12)

Zu einer Lösung von 85 mg der nach Beispiel 2 hergestellten Säure in 8 ml Methylenchlorid tropft man bei 0 °C eine etherische Diazomethanlösung bis zur bleibenden Gelbfärbung und rührt 15 Minuten bei 0 °C. Anschließend dampft man im Vakuum ein und chromatographiert den Rückstand an Kieselgel. Mit Hexan/Essigester (2+8) erhält man 69 mg der Titelverbindung als farbloses Öl.
- IR (Film):: 3600, 2925, 2855, 1738, 1658, 990 cm⁻¹

### Beispiel 16

### (+/-)-(5RS)-5-Hydroxy-5-[cis-(2RS)-2-(1E,3E)-(5S)-5-hydroxy-1,3-tridecadienyl)-(1RS)-cyclopentyl]-pentansäuremethylester Diastereomer pol (12)

In Analogie zu Beispiel 15 erhält man aus 120 mg der nach Beispiel 6 hergestellten Säure 102 mg der Titelverbindung als Öl.
- IR (Film):: 3610, 2925, 2855, 1737, 1660, 990 cm⁻¹

### Beispiel 17

### (+/-)-(5RS)-5-Acetoxy-5-[cis-(2RS)-2-(1E,3E)-(5S)-5-hydroxy-1,3-tridecadienyl)-(1RS)-cyclopentyl]-pentansäuremethylester Diastereomer pol (12)

In Analogie zu Beispiel 15 erhält man aus 80 mg der nach Beispiel 7 hergestellten Säure 71 mg der Titelverbindung als farbloses Öl.
- IR (Film):: 3400, 2924, 2858, 1739, 1658, 1240, 990 cm⁻¹

### Beispiel 18

### (+/-)-(5RS)-5-Hydroxy-5-[cis-(2RS)-2-(1E,3E)-(5S)-5-hydroxy-1,3-tridecadienyl-(1RS)-cyclopentyl]-pentansäure-tris-(hydroxymethyl)-aminomethansalz Diastereomer pol (12)

Zu einer Lösung von 40 mg der nach Beispiel 6 hergestellten Carbonsäure in 6 ml Acetonitril fügt man bei 70 °C eine Lösung von 15 mg Tris-(hydroxymethyl)-aminomethan in 0,03 ml Wasser. Man läßt unter Rühren abkühlen, decantiert nach 16 Stunden vom Lösungsmittel ab und trocknet den Rückstand im Vakuum. Man isoliert 14 mg der Titelverbindung als wachsartige Masse.

### Beispiel 19

### (+/-)-(5RS)-5-Hydroxy-5-[cis-(2RS)-2-(1E,3E)-(5S)-5-hydroxy-1,3-tridecadienyl)-(1RS)-cyclopentyl]-pentansäure-1,5-lacton Diastereomer pol (12)

Zu einer Lösung von 27 mg der nach Beispiel 6 hergestellten Carbonsäure in 5 ml Toluol fügt man bei 24 °C über einen Zeitraum von 24 Stunden portionsweise 0,5 g wasserfreies Magnesiumsulfat und rührt weitere 24 Stunden bei 24 °C. Anschließend filtriert man und chromatographiert den Eindampfrückstand an Kieselgel. Mit Toluol/Essigester (7+3) eluiert man 14 mg des 1,5-Lactons als farbloses Öl.
- IR:: 3600, 2930, 2860, 1728, 1250, 990 cm⁻¹

## Patentansprüche

1. Leukotrien B₄-Derivate der Formel I 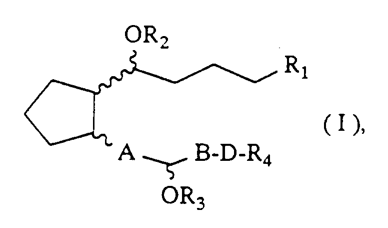 worin
R¹ CH₂OH, CH₃, CF₃, COOR⁵, CONR⁶R⁷ oder⁷
R¹ gemeinsam mit R² eine Carbonylgruppe bedeutet,
R² und R³ gleich oder verschieden, H oder ein organischer Säurerest mit 1-15 C-Atomen darstellen,
R⁴ H, (C₁-C₁₀) gegebenenfalls einfach oder mehrfach durch Chlor oder Brom substituierter Alkyl, C₃-C₁₀ Cycloalkyl, gegebenenfalls unabhängig voneinander einfach oder mehrfach durch Chlor, Brom, Phenyl C₁-C₄-Alkyl, C₁-C₄-Alkoxy, Fluormethyl, Chlormethyl, Trifluormethyl, Carboxy oder Hydroxy substituiertes C₆-C₁₀ Arylrest oder einen 5-6 gliedriger aromatischer heterocyclischen Ring mit wenigstens 1 Heteroatom symbolisieren,
R⁵ Wasserstoff, C₁-C₁₀ Alkyl, C₃-C₁₀ Cycloalkyl, gegebenenfalls durch 1-3 Chlor, Brom, Phenyl, C₁-C₄-Alkyl, C₁-C₄-Alkoxy, Fluormethyl, Chlormethyl, Trifluormethyl, Carboxy oder Hydroxy substituierter C₆-C₁₀ Arylrest, CH₂-CO-(C₆-C₁₀) Aryl oder ein 5-6 gliedriger Ring mit wenigstens 1 Heteroatom bedeutet,
A eine trans,trans-CH=CH-CH=CH, eine -CH₂CH₂-CH=CH- oder eine Tetramethylengruppe darstellt,
B eine C₁-C₁₀ grad- oder verzweigtkettige Alkylengruppe, die gegebenenfalls durch Fluor substituiert sein kann oder die Gruppe 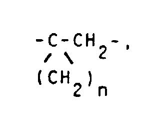 symbolisiert,
D eine Direktbindung, Sauerstoff, Schwefel, -C≡C-, -CH=CR⁸, oder gemeinsam mit
B auch eine Direktbindung bedeuten können,
R⁶ und R⁷ gleich oder verschieden sind und H oder C₁-C₄ Alkyl oder R⁷ H und R⁶ C₁-C₁₀-Alkanoyl oder C₁-C₁₀ Alkansulfonyl darstellen,
R⁸ H, C₁-C₅ Alkyl, Chlor, Brom bedeutet,
n 3-5 ist sowie, wenn R⁵ Wasserstoff bedeutet, deren Salze mit physiologisch verträglichcn Basen und deren Cyclodextrinclathrate.

2. Verfahren zur Herstellung von Leukotrien-B₄-Derivate der Formel I, 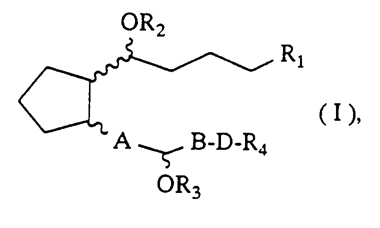 worin
R¹ CH2OH, CH₃, CF₃, COOR⁵, CONR⁶R⁷, oder
R¹ gemeinsam mit R² eine Carbonylgruppe bedeutet, R² und R³ gleich oder verschieden H oder ein organischer Säurerest mit 1-15C-Atomen darstellen,
R⁴ H, C₁-C₁₀ gegebenenfalls einfach oder mehrfach durch Chlor oder Brom substituierter Alkyl, C₃-C₁₀-Cycloalkyl, gegebenenfalls unabhängig voneinander einfach oder mehrfach durch Chlor, Brom, Phenyl, C₁-C₄-Alkyl, C₁-C₄-Alkoxy, Fluormethyl, Chlormethyl, Trifluormethyl, Carboxy oder Hydroxy substituierter C₆-C₁₀-Arylrest oder ein 5-6 gliedriger aromatischer heterocyclischer Ring mit wenigstens 1 Heteroatom symbolisieren,
R⁵ Wasserstoff, C₁-C₁₀ Alkyl, C₃-C₁₀ Cycloalkyl, gegebenenfalls durch 1-3 Chlor, Brom, Phenyl, C₁-C₄-Alkyl, C₁-C₄-Alkoxy, Fluormethyl, Chlormethyl, Trifluormethyl, Carboxy oder Hydroxy substituierter C₆-C₁₀ Arylrest, CH₂-CO-(C₆-C₁₀) Aryl oder ein 5-6 gliedriger Ring mit wenigstens 1 Heteroatom bedeutet,
A eine trans,trans-CH=CH-CH=CH, eine -CH₂CH₂-CH=CH- oder eine Tetramethylengruppe darstellt,
B eine C₁-C₁₀ grad- oder verzweigtkettige Alkylengruppe, die gegebenenfalls durch Fluor substituiert sein kann oder die Gruppe 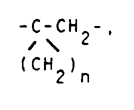 symbolisiert,
D eine Direktbindung, Sauerstoff, Schwefel, -C≡C-,-CH=CR⁸, oder gemeinsam mit
B auch eine Direktbindung bedeuten können,
R⁶ und R⁷ gleich oder verschieden sind und H oder C₁-C₄-Alkyl oder R⁷ H und R⁶ C₁-C₁₀-Alkanoyl oder C₁-C₁₀ Alkansulfonyl darstellen,
R⁸ H, C₁-C₅ Alkyl, Chlor, Brom bedeutet,
n 3-5 ist sowie, wenn R⁵ Wasserstoff bedeutet, deren Salze mit physiologisch vertraglichen Basen und deren Cyclodextrinclathrate, dadurch gekennzeichnet, daß man einen Aldehyd der Formel II 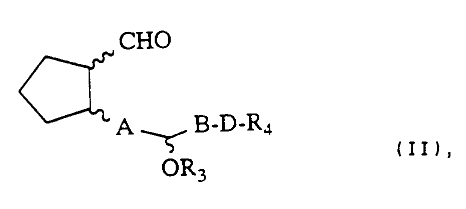
worin A, B, D, R³ und R⁴ die oben angegebene Bedeutung haben, gegebenenfalls nach Schutz freier Hydroxygruppen mit einer magnesiumorganischen Verbindung der Formel III,
X-Mg-CH₂-CH₂-CH₂-CH₂-R⁹ (III),
worin X Chlor, Brom oder Iod und R⁹ -CH₃, CF₃ oder -OR¹⁰ darstellt, worin R₁₀ einen leicht abspaltbaren Etherrest bedeuten, umsetzt und gegebenenfalls anschließend in beliebiger Reihenfolge Isomere trennt, geschützte Hydroxygruppen freisetzt und/oder eine freie Hydroxygruppe verestert und/oder die 1-Hydroxygruppe zur Carbonsäure oxidiert und/oder Doppelbindungen hydriert und/oder eine veresterte Carboxylgruppe (R¹=COOR⁵) verseift und/oder reduziert und/oder eine Carboxylgruppe (R⁵=H) verestert und/oder eine freie Carboxylgruppe (R⁵=H) in ein Amid (R¹=CONHR⁶R⁷) überführt oder eine Carboxylgruppe mit einer physiologisch verträglichen Base in ein Salz überführt.

3. Pharmazeutische Zubereitungen, die eine oder mehrere Verbindungen nach Anspruch 1 enthalten.

## Claims

1. Leukotriene B₄ derivatives of formula I 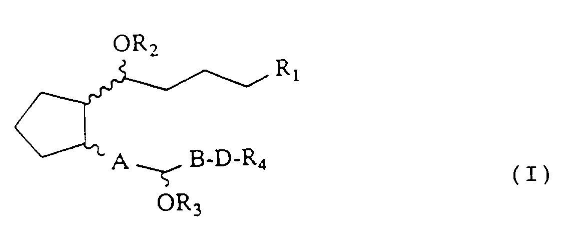 wherein
R¹ represents CH₂OH, CH₃, CF₃, COOR⁵, CONR⁶R⁷ or
R¹ together with R² represents a carbonyl group,
R² and R³, which are the same or different, each represents H or an organic acid radical having from 1 to 15 carbon atoms,
R⁴ represents H, (C₁-C₁₀)alkyl optionally mono- or poly-substituted by chlorine or by bromine, C₃-C₁₀cycloalkyl, C₆-C₁₀aryl optionally mono- or poly-substituted independently from one another by chlorine, bromine, phenyl, C₁-C₄alkyl, C₁-C₄alkoxy, fluoromethyl, chloromethyl, trifluoromethyl, carboxy or hydroxy, or a 5- or 6-membered aromatic heterocyclic ring having at least 1 hetero atom,
R⁵ represents hydrogen, C₁-C₁₀alkyl, C₃-C₁₀cycloalkyl, C₆-C₁₀aryl optionally substituted by from 1 to 3 substituents chlorine, bromine, phenyl, C₁-C₄alkyl, C₁-C₄alkoxy, fluoromethyl, chloromethyl, trifluoromethyl, carboxy or hydroxy, CH₂-CO-(C₆-C₁₀)aryl or a 5- or 6-membered ring having at least 1 hetero atom,
A represents a trans,trans-CH=CH-CH=CH, a -CH₂CH₂-CH=CH- or a tetramethylene group,
B represents a straight-chain or branched C₁-C₁₀alkylene group that may optionally be substituted by fluorine, or represents the group 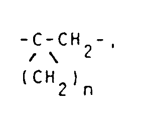
D represents a direct bond, oxygen, sulphur, -C≡C-, -CH=CR⁸ or, together with
B may also represent a direct bond,
R⁶ and R⁷ are the same or different and each represents H or C₁-C₄alkyl or R⁷ represents H and R⁶ represents C₁-C₁₀alkanoyl or C₁-C₁₀alkanesulphonyl,
R⁸ represents H, C₁-C₅alkyl, chlorine or bromine,
n is 3 to 5 and, when R⁵ represents hydrogen, the salts thereof with physiologically tolerable bases and the cyclodextrin clathrates thereof.

2. Process for the preparation of leukotriene B₄ derivatives of formula I, 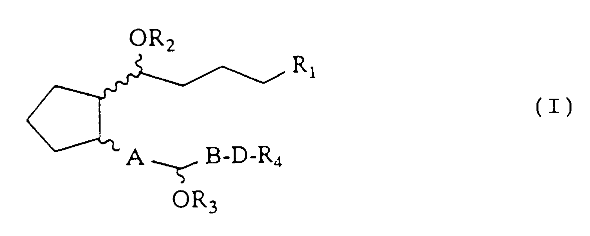 wherein
R¹ represents CH₂OH, CH₃, CF₃, COOR⁵, CONR⁶R⁷ or
R¹ together with R² represents a carbonyl group,
R² and R³, which are the same or different, each represents H or an organic acid radical having from 1 to 15 carbon atoms,
R⁴ represents H, C₁-C₁₀alkyl optionally mono- or polysubstituted by chlorine or by bromine, C₃-C₁₀cycloalkyl, C₆-C₁₀aryl optionally mono- or poly-substituted independently from one another by chlorine, bromine, phenyl, C₁-C₄alkyl, C₁-C₄alkoxy, fluoromethyl, chloromethyl, trifluoromethyl, carboxy or hydroxy, or represents a 5- or 6-membered aromatic heterocyclic ring having at least 1 hetero atom,
R⁵ represents hydrogen, C₁-C₁₀alkyl, C₃-C₁₀cycloalkyl, C₆-C₁₀aryl optionally substituted by from 1 to 3 substituents chlorine, bromine, phenyl, C₁-C₄alkyl, C₁-C₄alkoxy, fluoromethyl, chloromethyl, trifluoromethyl, carboxy or hydroxy, CH₂-CO-(C₆-C₁₀)aryl or a 5- or 6- membered ring having at least 1 hetero atom,
A represents a trans,trans-CH=CH-CH=CH, a -CH₂CH₂-CH=CH- or a tetramethylene group,
B represents a straight-chain or branched C₁-C₁₀alkylene group that may optionally be substituted by fluorine, or represents the group 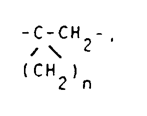
D represents a direct bond, oxygen, sulphur -C≡C-, -CH=CR⁸ or, together with
B may also represent a direct bond,
R⁶ and R⁷ are the same or different and each represents H or C₁-C₄alkyl or R⁷ represents H and R⁶ represents C₁-C₁₀alkanoyl or C₁-C₁₀alkanesulphonyl,
R⁸ represents H, C₁-C₅alkyl, chlorine or bromine,
n is 3 to 5 and, when R⁵ represents hydrogen, the salts thereof with physiologically tolerable bases and the cyclodextrin clathrates thereof, characterised in that an aldehyde of formula II 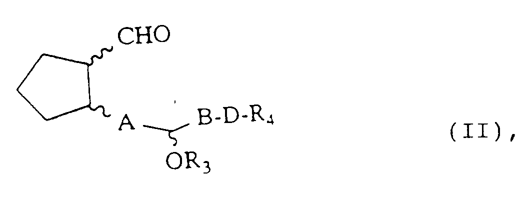
wherein A, B, D, R³ and R⁴ are as defined above, is reacted, optionally after the protection of free hydroxy groups, with an organomagnesium compound of formula III
X-Mg-CH₂-CH₂-CH₂-CH₂-R⁹ (III),
wherein X represents chlorine, bromine or iodine and R⁹ represents -CH₃, CF₃ or -OR¹⁰ in which R₁₀ represents a readily removable ether radical and, where appropriate then, in any sequence, isomers are separated, protected hydroxy groups are freed and/or a free hydroxy group is esterified and/or a 1-hydroxy group is oxidized to carboxylic acid and/or double bonds are hydrogenated and/or an esterified carboxy group (R¹=COOR⁵) is hydrolysed and/or reduced and/or a carboxy group (R⁵=H) is esterified and/or a free carboxy group (R⁵=H) is converted into an amide (R¹=CONHR⁶R⁷) or a carboxy group is converted into a salt with a physiologically tolerable base.

3. Pharmaceutical compositions that comprise one or more compounds according to claim 1.

## Revendications

1. Dérivés du leucotriène-B₄ de formule I ci-dessous 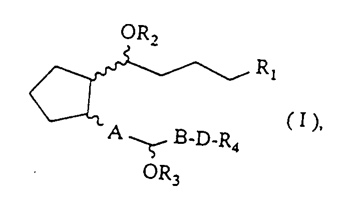 formule dans laquelle
R¹ désigne CH₂OH, CH₃, CF₃, COOR⁵ ou CONR⁶R⁷, ou bien
R¹ forme avec R² un groupe carbonyle,
R² et R³, qui peuvent être identiques ou différents l'un de l'autre, désignent l'hydrogène ou un radical d'acide organique pouvant avoir de 1 à 15 atomes de carbone,
R⁴ désigne l'hydrogène, un alkyle en C₁-C₁₀ éventuellement substitué par un ou plusieurs atomes de chlore ou de brome ou un cycloalkyle en C₃-C₁₀, un aryle en C₆-C₁₀ éventuellement substitué, d'une manière indépendante, par un ou plusieurs atomes de chlore ou de brome ou groupes phényle, alkyles en C₁-C₄, alcoxy en C₁-C₄, fluorométhyle, chlorométhyle, trifluorométhyle, carboxy ou hydroxy, ou encore un radical hétérocyclique aromatique à 5 ou 6 chaînons avec au moins un hétéroatome,
R⁵ étant l'hydrogène, un alkyle en C₁-C₁₀ ou un cycloalkyle en C₃-C₁₀, un radical aryle en C₆-C₁₀ éventuellement substitué par 1 à 3 atomes de chlore ou de brome ou groupes phényle, alkyles en C₁-C₄, alcoxy en C₁-C₄, fluorométhyle, chlorométhyle, trifluorométhyle, carboxy ou hydroxy, un CH₂-CO-(aryle en C₆-C₁₀) ou encore un radical cyclique avec 5 ou 6 ayant au moins un hétéroatome,
A désigne un groupe trans,trans-CH=CH-CH=CH-, un groupe -CH₂CH₂-CH=CH- ou un groupe tétraméthylène,
B un groupe alkylène en C₁-C₁₀ linéaire ou ramifié pouvant être éventuellement substitué par du fluor ou un groupe 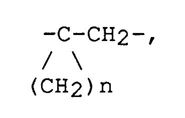 et
D une liaison directe, l'oxygène, le soufre ou un groupe -C≡C- ou -CH=CR⁸, ou bien avec
B également une liaison directe,
R⁶ et R⁷, identiques ou différents l'un de l'autre, étant l'hydrogène ou des alkyles en C₁-C₄ ou bien R⁷ étant l'hydrogène et R⁶ un alcanoyle en C₁-C₁₀ ou un alcanesulfonyle en C₁-C₁₀,
R⁸ l'hydrogène, un alkyle en C₁-C₅, le chlore ou le brome et
n un nombre de 3 à 5, ainsi que, si R⁵ est l'hydrogène, des sels de ces dérivés avec des bases physiologiquement compatibles, et leur clathrates de cyclodextrines.

2. Procédé de préparation des dérivés du leucotriène-B₄ de formule I 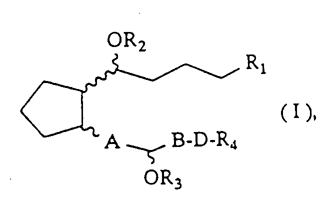 dans laquelle
R¹ désigne CH₂OH, CH₃, CF₃, COOR⁵ ou CONR⁶R⁷, ou bien
R¹ forme avec R² un groupe carbonyle,
R² et R³, qui peuvent être identiques ou différents l'un de l'autre, désignent l'hydrogène ou un radical d'acide organique pouvant avoir de 1 à 15 atomes de carbone,
R⁴ désigne l'hydrogène, un alkyle en C₁-C₁₀ éventuellement substitué par un ou plusieurs atomes de chlore ou de brome, un cycloalkyle en C₃-C₁₀, un radical aryle en C₆-C₁₀ éventuellement substitué, d'une manière indépendante, par un ou plusieurs atomes de chlore ou de brome ou groupes phényle, alkyles en C₁-C₄, alcoxy en C₁-C₄, fluorométhyle, chlorométhyle, trifluorométhyle, carboxy ou hydroxy, ou encore un radical hétérocyclique aromatique à 5 ou 6 chaînons avec au moins un hétéroatome,
R⁵ étant l'hydrogène, un alkyle en C₁-C₁₀ ou un cycloalkyle en C₃-C₁₀, un radical aryle en C₆-C₁₀ éventuellement substitué par 1 à 3 atomes de chlore ou de brome ou groupes phényle, alkyles en C₁-C₄, alcoxy en C₁-C₄, fluorométhyle, chlorométhyle, trifluorométhyle, carboxy ou hydroxy, un CH₂-CO-(aryle en C₆-C₁₀) ou encore un radical cyclique avec 5 ou 6 chaînons ayant au moins un hétéroatome,
A désigne un groupe trans,trans-CH=CH-CH=CH-, un groupe -CH₂CH₂-CH=CH- ou un groupe tétraméthylène,
B un groupe alkylène en C₁-C₁₀ linéaire ou ramifié pouvant être éventuellement substitué par du fluor ou un groupe 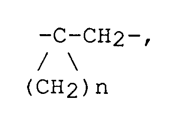 et
D une liaison directe, l'oxygène, le soufre ou un groupe -C≡C- ou -CH=CR⁸, ou bien avec
B également une liaison directe,
R⁶ et R⁷, identiques ou différents l'un de l'autre, étant l'hydrogène ou des alkyles en C₁-C₄ ou bien R⁷ étant l'hydrogène et R⁶ un alcanoyle en C₁-C₁₀ ou un alcanesulfonyle en C₁-C₁₀,
R⁸ l'hydrogène, un alkyle en C₁-C₅, le chlore ou le brome,
n un nombre de 3 à 5, ainsi que, si R⁵ est l'hydrogène, des sels de ces dérivés avec des bases physiologiquement compatibles et leur clathrates de cyclodextrines.
procédé caractérisé en ce que l'on fait réagir un aldéhyde de formule II 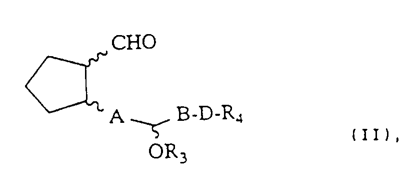 dans laquelle A, B, D, R³ et R⁴ ont les mêmes significations données ci-dessus, éventuellement après protection de groupes hydroxyliques libres, avec un composé organomagnésien de formule III
X-Mg-CH₂-CH₂-CH₂-CH₂-R⁹ (III),
dans laquelle X désigne le chlore, le brome ou l'iode et R⁹ un groupe -CH₃, -CF₃ ou -OR¹⁰, R¹⁰ étant un radical d'éther facilement éliminable, puis le cas échéant, et en procédant dans un ordre quelconque, on sépare les isomères, on libère les groupes hydroxyliques protégés et/ou on estérifie un groupe hydroxylique libre et/ou on oxyde le groupe 1-hydroxylique en groupe carboxylique et/ou on hydrogène des doubles liaisons et/ou on saponifie un groupe carboxylique estérifié (R¹ = COOR⁵) et/ou on réduit et/ou on estérifie un groupe carboxylique (R⁵ = H) et/ou on transforme un groupe carboxylique libre (R⁵ = H) en un amide (R¹ = CONHR⁶R⁷) ou encore on salifie un groupe carboxylique avec une base physiologiquement compatible.

3. Préparations pharmaceutiques qui comprennent un ou plusieurs composés de la revendication 1.
